(19) Europäisches Patentamt
European Patent Office
Office européen des brevets

(11) EP 0 812 835 B1

(12) EUROPEAN PATENT SPECIFICATION

(45) Date of publication and mention of the grant of the patent:
**23.05.2001 Bulletin 2001/21**

(21) Application number: **96904292.8**

(22) Date of filing: **01.03.1996**

(51) Int Cl.[7]: **C07D 277/14**, C07D 263/24, A61K 31/42, A61K 31/425, A61K 31/44

(86) International application number:
**PCT/JP96/00487**

(87) International publication number:
**WO 96/26931 (06.09.1996 Gazette 1996/40)**

# (54) OPTICALLY ACTIVE THIAZOLIDINONE DERIVATIVES

OPTISCH AKTIVE THIAZOLIDINONDERIVATE

DERIVES DE THIAZOLIDINONE OPTIQUEMENT ACTIFS

(84) Designated Contracting States:
**AT BE CH DE DK ES FI FR GB GR IE IT LI LU MC NL PT SE**

(30) Priority: **02.03.1995 JP 4223795**
**27.10.1995 JP 27995195**

(43) Date of publication of application:
**17.12.1997 Bulletin 1997/51**

(73) Proprietor: **SANKYO COMPANY LIMITED**
**Chuo-ku, Tokyo 103-0023 (JP)**

(72) Inventors:
- **ISHIHARA, Sadao**
  **Shinagawa-ku Tokyo 140 (JP)**
- **SAITO, Fujio**
  **Shinagawa-ku Tokyo 140 (JP)**
- **OHHATA, Yasuo**
  **Shinagawa-ku Tokyo 140 (JP)**
- **MIYAKE, Shigeki**
  **Shinagawa-ku Tokyo 140 (JP)**
- **YORIKANE, Ryosuke**
  **Shinagawa-ku Tokyo 140 (JP)**
- **FUKUDA, Norio**
  **Shinagawa-ku Tokyo 140 (JP)**
- **TABATA, Keiichi**
  **Shinagawa-ku Tokyo 140 (JP)**
- **MAKINO, Mitsuko**
  **Shinagawa-ku Tokyo 140 (JP)**

(74) Representative: **Andrews, Timothy Stephen**
**Marks & Clerk,**
**57-60 Lincoln's Inn Fields**
**London WC2A 3LS (GB)**

(56) References cited:
**JP-A- 2 028 167** **JP-A- 5 213 910**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 812 835 B1

## Description

[Technical field]

The present invention relates to optically active thiazolidinone derivatives having an excellent collateral vessel dilating action and an anti-angina pectoris action, a composition for prevention or therapy of angina pectoris comprising them as an active ingredient, use of them for producing a pharmaceutical preparation for prevention or therapy of angina pectoris, or a preparation process thereof; or a composition for prevention or therapy of an ulcerative disease comprising thia- or oxazolidinone derivatives as an active ingredient, use of them for producing a pharmaceutical preparation for prevention or therapy of an ulcerative disease.

[Prior art]

At present, as a therapeutic agent for cardiovascular diseases, particularly for angina pectoris, nitroglycerin is most frequently used clinically. However, nitroglycerin easily undergoes the first-pass effect and has a defect that duration of its action is short. Meanwhile, headache, vertigo and tachycardia due to reduction in blood pressure appear as side effects. In view of such background, there has been demanded a therapeutic agent for angina pectoris with prolonged actions which clinically does not undergo the first-pass effect.

The present inventors have found, as a means for solving the above problem, a compound having a thia- or oxazolidinone skeleton, for example, compound A having the following formula:

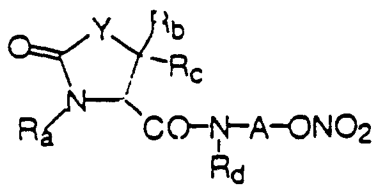

compound A

(wherein Ra, Rb, Rc and Rd represent a hydrogen atom, etc., A represents a $C_2$-$C_6$ alkylene group and Y represents an oxygen atom or a sulfur atom) (for example, Japanese Unexamined Patent Publication (KOKAI) No. Hei 5-213910). However, an anti-ulcerative action of these compounds has not been known at all.

[Disclosure of the invention]

The present inventors made further studies and have found that compounds having an optically active thiazolidinone skeleton have an excellent collateral vessel dilating action which is prolonged and exhibit less side effects, the compounds are useful as a preventive agent or a therapeutic agent for angina pectoris (particularly a therapeutic agent for angina pectoris) and the compounds have excellent stability to accomplish the present invention. Moreover, the present inventors made studies on pharmacological effects of the compounds having a thia- or oxazolidinone skelton and have also found that these compounds have an excellent anti-ulcerative action and the compounds are useful as a preventive agent or a therapeutic agent for an ulcerative disease (particularly a therapeutic agent for an ulcerative disease).

The present invention provides optically active thiazolidinone derivatives, a composition for prevention or therapy of angina pectoris comprising them as an active ingredient, use of them for producing a pharmaceutical preparation for prevention or therapy of angina pectoris, or a preparation process thereof; or a composition for prevention or therapy of an ulcerative disease comprising thia- or oxazolidinone derivatives as an active ingredient, or use of them for producing a pharmaceutical preparation for prevention or therapy of an ulcerative disease.

[Constitution of the invention]

The optically active thiazolidinone derivatives of the present invention have the general formula:

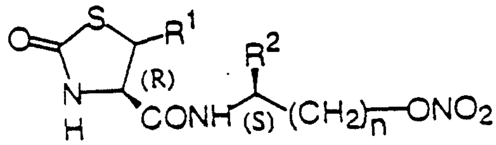

(I)

In the above formula,

$R^1$ represents a hydrogen atom, a $C_1$-$C_4$ alkyl group, a phenyl group, a substituted phenyl group (the substituent represents $C_1$-$C_4$ alkyl, $C_1$-$C_4$ alkoxy or halogen), a phenyl-$C_1$-$C_2$ alkyl group or a substituted phenyl-$C_1$-$C_2$ alkyl group (the substituent of the phenyl represents $C_1$-$C_4$ alkyl, $C_1$-$C_4$ alkoxy or halogen);
$R^2$ represents a $C_1$-$C_6$ alkyl group; and
n represents 1 or 2.

The thia- or oxazolidinone derivatives which are an active ingredient of a preventive agent or a therapeutic agent for an ulcerative disease have the general formula:

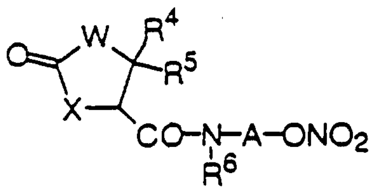

(II)

In the above formula,

W represents a sulfur atom or an oxygen atom and X represents a group having the formula: -N($R^3$)- or X represents a sulfur atom or an oxygen atom and W represents a group having the formula: -N($R^3$)-;
$R^3$ represents a hydrogen atom, a $C_1$-$C_6$ alkyl group or an aryl-$C_1$-$C_4$ alkyl group;
$R^4$ and $R^5$ may be the same or different and represents a hydrogen atom, a $C_1$-$C_6$ alkyl group, an aryl-$C_1$-$C_4$ alkyl group, an aryl group, a 5-or 6-membered aromatic heterocyclic group containing 1 to 3 hetero atoms selected from the group consisting of nitrogen atoms, oxygen atoms and sulfur atoms, which may be optionally condensed with a benzene ring or a 5- or 6-membered aromatic heterocyclic group containing 1 to 3 hetero atoms selected from the group consisting of nitrogen atoms, oxygen atoms and sulfur atoms which may be optionally substituted and condensed with a benzene ring (the substituent represents $C_1$-$C_6$ alkyl, amino, mono-$C_1$-$C_6$ alkylamino or di-$C_1$-$C_6$ alkylamino);
$R^6$ represents a hydrogen atom, a $C_1$-$C_6$ alkyl group or an aryl-$C_1$-$C_4$ alkyl group;
A represents a $C_2$-$C_6$ alkylene group or a substituted $C_2$-$C_6$ alkylene group (the substituent represents a carboxyl group, a $C_1$-$C_6$ alkoxycarbonyl group or an aryloxycarbonyl group); and
the above-mentioned aryl represents $C_6$-$C_{10}$ aryl or substituted $C_6$-$C_{10}$ aryl (the substituent represents $C_1$-$C_6$ alkyl, $C_1$-$C_6$ alkoxy, hydroxy, halogen, amino, mono-$C_1$-$C_6$ alkylamino, di-$C_1$-$C_6$ alkylamino or nitro).

The $C_1$-$C_4$ alkyl group of $R^1$ etc., or the alkyl moiety of the $C_1$-$C_4$ alkoxy group included in $R^1$ may include, for example, a methyl, ethyl, propyl, isopropyl, butyl, s-butyl, isobutyl and t-butyl group, preferably a $C_1$-$C_3$ alkyl group, more preferably a $C_1$-$C_2$ alkyl group, and particularly preferably a methyl group.
The halogen atom included in $R^1$ may include, for example, a fluorine, chlorine, bromine and iodine atom, and preferably a fluorine atom or a chlorine atom.
The phenyl-$C_1$-$C_2$ alkyl group of $R^1$ may include preferably benzyl group or phenethyl group, and more preferably benzyl group.
The $C_1$-$C_6$ alkyl group of $R_2$ may include, for example, a methyl, ethyl, propyl, isopropyl, butyl, s-butyl, isobutyl, t-butyl, pentyl and hexyl group, preferably a $C_1$-$C_4$ alkyl group, more preferably a methyl, ethyl, propyl, isopropyl, butyl or isobutyl group, still more preferably a methyl, propyl, butyl or isobutyl group, and particularly preferably a methyl group.
The $C_1$-$C_6$ alkyl group of $R^3$, $R^4$, $R^5$, $R^6$, etc., or the alkyl moiety of the $C_1$-$C_6$ alkoxy group or the $C_1$-$C_6$ alkylamino group included in $R^3$, A, etc., may include the above group, preferably a $C_1$-$C_4$ alkyl group, more preferably

a $C_1$-$C_2$ alkyl group, and particularly preferably a methyl group.

The aryl moiety of the aryl-$C_1$-$C_4$ alkyl group of $R^3$, $R^4$, $R^5$ and $R^6$ may include the group described below and the alkyl moiety may include the above group, and may include, for example, a benzyl, phenethyl, 2-phenylpropyl, 3-phenylpropyl, 4-phenylbutyl, diphenylmethyl, 1-naphthylmethyl and 2-naphthylmethyl group, preferably phenyl-($C_1$-$C_4$ alkyl) group, more preferably a benzyl group or a phenethyl group, and particularly preferably a benzyl group.

The aryl group of $R^4$ and $R^5$ or the aryl moiety of the aryloxycarbonyl group included in A may include the above group, and preferably a phenyl group.

The halogen of the substituent of the aryl group of $R^4$ and $R^5$ may include the above group, and preferably a fluorine atom or a chlorine atom.

The 5- or 6-membered aromatic heterocyclic group containing 1 to 3 hetero atoms selected from the group consisting of nitrogen atoms, oxygen atoms and sulfur atoms which may be optionally condensed with a benzene ring may include, for example, furyl, thienyl, pyrrolyl, oxazolyl, isoxazolyl, thiazolyl, isothiazolyl, imidazolyl, pyrazolyl, pyridyl, pyridazinyl, pyrimidinyl, indolyl, quinolyl and quinazolinyl, preferably furyl, thienyl, oxazolyl, isoxazolyl, thiazolyl, isothiazolyl or pyridyl, more preferably a furyl, thienyl or pyridyl group, and particularly preferably a furyl group or a thienyl group.

The alkylene moiety of the $C_2$-$C_6$ alkylene group of A may include, for example, ethylene, methylethylene, trimethylene, tetramethylene, pentamethylene and hexamethylene, preferably a $C_2$-$C_4$ alkylene group, and particularly preferably an ethylene group or a methylethylene group.

In the compound (II), those containing a carboxy or phenol moiety may form salts with a base. Such salts may include, for example, a salt with an alkali metal such as lithium, sodium and potassium, a salt with an alkaline earth metal such as barium and calcium, a salt with other metals such as magnesium and aluminum, a salt with an organic amine such as dicyclohexylamine and a salt with a basic amino acid such as lysine and arginine, and preferably a salt with an alkali metal. Meanwhile, the compound (II) containing amino or alkylamino moieties can form salts with an acid. Such salts may include, for example, a salt with an inorganic acid such as hydrochloric acid, bromic acid, sulfuric acid, phosphoric acid and carbonic acid, a salt with a carboxylic acid such as acetic acid, fumaric acid, maleic acid, oxalic acid, malonic acid, succinic acid, citric acid, malic acid and benzoic acid, a salt with a sulfonic acid such as methanesulfonic acid, ethanesulfonic acid, benzenesulfonic acid and toluenesulfonic acid and a salt with an acidic amino acid such as glutamic acid and aspartic acid, and preferably a salt with hydrochloric acid or a carboxylic acid.

Further, the present invention includes hydrates of the compound (I) and in the case where an asymmetric carbon exists in a molecule of the compound (II), the present invention includes a racemic modification and an optically active substance and also includes the compound (II) or hydrates of a salt thereof.

Meanwhile, the compound (I) included in the compound (II) also has an excellent anti-ulcerative action.

The compound having the general formula (I) may include preferably

1) a compound in which $R^1$ is a hydrogen atom, a $C_1$-$C_4$ alkyl group, a phenyl group, a substituted phenyl group (the substituent is methyl, methoxy, fluorine or chlorine), a benzyl group, a substituted benzyl group (the substituent is methyl, methoxy, fluorine or chlorine), a phenethyl group or a substituted phenethyl group (the substituent is methyl, methoxy, fluorine or chlorine),
2) a compound in which $R^1$ is a hydrogen atom, a methyl group, a 4-methoxyphenyl group or a benzyl group,
3) a compound in which $R^1$ is a hydrogen atom,
4) a compound in which $R^2$ is a $C_1$-$C_4$ alkyl group,
5) a compound in which $R^2$ is a methyl group, an ethyl group, a propyl group, an isopropyl group, a butyl group or an isobutyl group,
6) a compound in which $R^2$ is a methyl group, a propyl group, a butyl group or an isobutyl group,
7) a compound in which $R^2$ is a methyl group, and
8) a compound in which n is 1.

Meanwhile, an optional combination of the compound selected arbitrarily from the group consisting of 1)-3), 4)-7) and 8) is preferred and may include, for example, the following compounds.

9) a compound in which $R^1$ is a hydrogen atom, a $C_1$-$C_4$ alkyl group, a phenyl group, a substituted phenyl group (the substituent is methyl, methoxy, fluorine or chlorine), a benzyl group, a substituted benzyl group (the substituent is methyl, methoxy, fluorine or chlorine), a phenethyl group or a substituted phenethyl group (the substituent is methyl, methoxy, fluorine or chlorine);

$R^2$ is a $C_1$-$C_4$ alkyl group; and
n is 1,

10) a compound in which $R^1$ is a hydrogen atom, a methyl group, a 4-methoxyphenyl group or a benzyl group;

$R^2$ is a methyl group, an ethyl group, a propyl group, an isopropyl group, a butyl group or an isobutyl group; and n is 1,

11) a compound in which $R^1$ is a hydrogen atom;

$R^2$ is a methyl group, a propyl group, a butyl group or an isobutyl group; and n is 1, and

12) a compound in which $R^1$ is a hydrogen atom; and
$R^2$ is a methyl group.

Meanwhile, the compound having the above general formula (II) may include preferably

13) a compound in which $R^3$ is a hydrogen atom, a $C_1$-$C_4$ alkyl group, a benzyl group or a phenethyl group;
14) a compound in which $R^3$ is a hydrogen atom, a methyl group or a benzyl group,
15) a compound in which W is a sulfur atom or an oxygen atom and X is a group having the formula: -N($R^3$)- (wherein $R^3$ is a hydrogen atom) or
X is a sulfur atom and W is a group having the formula: -N($R^3$)-(wherein $R^3$ is a hydrogen atom),
16) a compound in which W is a sulfur atom or an oxygen atom and X is a group having the formula: -N$R^3$- (wherein $R^3$ is a hydrogen atom),
17) a compound in which $R^4$ and $R^5$ may be the same or different and each is a hydrogen atom, a $C_1$-$C_4$ alkyl group, a phenyl-$C_1$-$C_4$ alkyl group, a substituted phenyl-$C_1$-$C_4$ alkyl group (the substituent of the phenyl is $C_1$-$C_4$ alkyl, $C_1$-$C_4$ alkoxy, hydroxy, halogen or nitro), a naphthylmethyl group, a phenyl group, a substituted phenyl group (the substituent is $C_1$-$C_4$ alkyl, $C_1$-$C_4$ alkoxy, hydroxy, halogen or nitro), a naphthyl group or an unsubstituted or a $C_1$-$C_4$ alkyl-substituted furyl, thienyl, pyridyl, oxazolyl, thiazolyl, isoxazolyl or isothiazolyl group,
18) a compound in which $R^4$ and $R^5$ may be the same or different and each is a hydrogen atom, a methyl group, a benzyl group, a substituted benzyl group (the substituent is methyl, methoxy, hydroxy, fluoro or chloro), a phenethyl group, a substituted phenethyl group (the substituent is methyl, methoxy, hydroxy, fluoro or chloro), a phenyl group, a substituted phenyl group (the substituent is methyl, methoxy, hydroxy, fluoro or chloro), a furyl group, a thienyl group or a pyridyl group,
19) a compound in which $R^4$ is a hydrogen atom, a methyl group, a benzyl group, a substituted benzyl group (the substituent is methyl, methoxy or hydroxy), a phenyl group or a substituted phenyl group (the substituent is methyl, methoxy or hydroxy); and
$R^5$ is a hydrogen atom,
20) a compound in which $R^4$ is a hydrogen atom, a methyl group, a benzyl group, a phenyl group or a methoxyphenyl group; and
$R^5$ is a hydrogen atom,
21) a compound in which $R^4$ is a hydrogen atom, a methyl group, a benzyl group or a 4-methoxyphenyl group; and
$R^5$ is a hydrogen atom,
22) a compound in which $R^4$ is a hydrogen atom; and
$R^5$ is a hydrogen atom,
23) a compound in which $R^6$ is a hydrogen atom, a $C_1$-$C_4$ alkyl group, a benzyl group or a phenethyl group,
24) a compound in which $R^6$ is a hydrogen atom, a methyl group or a benzyl group,
25) a compound in which $R^6$ is a hydrogen atom,
26) a compound in which A is a $C_2$-$C_4$ alkylene group, a carboxy-$C_2$-$C_4$ alkylene group or a $C_1$-$C_4$ alkoxycarbonyl-$C_2$-$C_4$ alkylene group,
27) a compound in which A is a $C_2$-$C_4$ alkylene group, or
28) a compound in which A is an ethylene group or a 1-methylethylene group.

Meanwhile, an optional combination of the compound selected arbitrarily from the group consisting of 13)-16), 17)-22), 23)-25) and 26)-28) is also preferred and may include, for example, the following compounds:

29) a compound in which $R^3$ is a hydrogen atom, a $C_1$-$C_4$ alkyl group, a benzyl group or a phenethyl group;

$R^4$ and $R^5$ may be the same or different and each is a hydrogen atom, a $C_1$-$C_4$ alkyl group, a phenyl-$C_1$-$C_4$ alkyl group, a substituted phenyl-$C_1$-$C_4$ alkyl group (the substituent of the phenyl is $C_1$-$C_4$ alkyl, $C_1$-$C_4$ alkoxy, hydroxy, halogen or nitro), a naphthylmethyl group, a phenyl group, a substituted phenyl group (the substituent is $C_1$-$C_4$ alkyl, $C_1$-$C_4$ alkoxy, hydroxy, halogen or nitro), a naphthyl group or an unsubstituted or a $C_1$-$C_4$ alkyl-substituted furyl, thienyl, pyridyl, oxazolyl, thiazolyl, isoxazolyl or isothiazolyl group;
$R^6$ is a hydrogen atom, a $C_1$-$C_4$ alkyl group, a benzyl group or a phenethyl group; and
A is a $C_2$-$C_4$ alkylene group, a carboxy-$C_2$-$C_4$ alkylene group or a $C_1$-$C_4$ alkoxycarbonyl-$C_2$-$C_4$ alkylene group,

30) a compound in which $R^3$ is a hydrogen atom, a methyl group or a benzyl group;

$R^4$ and $R^5$ may be the same or different and each is a hydrogen atom, a methyl group, a benzyl group, a substituted benzyl group (the substituent is methyl, methoxy, hydroxy, fluoro or chloro), a phenethyl group, a substituted phenethyl group (the substituent is methyl, methoxy, hydroxy, fluoro or chloro), a phenyl group, a substituted phenyl group (the substituent is methyl, methoxy, hydroxy, fluoro or chloro), a furyl group, a thienyl group or a pyridyl group;
$R^6$ is a hydrogen atom, a methyl group or a benzyl group; and
A is a $C_2$-$C_4$ alkylene group,

31) a compound in which W is a sulfur atom or an oxygen atom and X is a group having the formula: -$NR^3$- (wherein $R^3$ is a hydrogen atom) or X is a sulfur atom and W is a group having the formula: -$NR^3$- (wherein $R^3$ is a hydrogen atom);

$R^4$ is a hydrogen atom, a methyl group, a benzyl group, a substituted benzyl group (the substituent is methyl, methoxy or hydroxy), a phenyl group or a substituted phenyl group (the substituent is methyl, methoxy or hydroxy);
$R^5$ is a hydrogen atom;
$R^6$ is a hydrogen atom; and
A is a $C_2$-$C_4$ alkylene group,

32) a compound in which W is a sulfur atom or an oxygen atom and X is a group having the formula: -$NR^3$- (wherein $R^3$ is a hydrogen atom);

$R^4$ is a hydrogen atom, a methyl group, a benzyl group, a phenyl group or a methoxyphenyl group;
$R^5$ is a hydrogen atom;
$R^6$ is a hydrogen atom; and
A is a $C_2$-$C_4$ alkylene group,

33) a compound in which W is a sulfur atom and X is a group having the formula: -$NR^3$- (wherein $R^3$ is a hydrogen atom);

$R^4$ is a hydrogen atom, a methyl group, a benzyl group or a 4-methoxyphenyl group;
$R^5$ is a hydrogen atom;
$R^6$ is a hydrogen atom; and
A is an ethylene group or a 1-methylethylene group, or

34) a compound in which W is a sulfur atom and X is a group having the formula: -$NR^3$- (wherein $R^3$ is a hydrogen atom);

$R^4$ is a hydrogen atom;
$R^5$ is a hydrogen atom;
$R^6$ is a hydrogen atom; and
A is an ethylene group or a 1-methylethylene group.

The preferred compound in the general formula (I) can be specifically exemplified in Table 1.

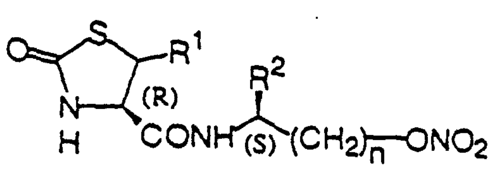

(I)

[Table 1]

| Compound No. | $R^1$ | $R^2$ | n |
|---|---|---|---|
| 1-1 | H | Me | 1 |
| 1-2 | Me | Me | 1 |
| 1-3 | Et | Me | 1 |
| 1-4 | Ph | Me | 1 |
| 1-5 | 4-Me-Ph | Me | 1 |
| 1-6 | 4-MeO-Ph | Me | 1 |
| 1-7 | 4-F-Ph | Me | 1 |
| 1-8 | 4-Cl-Ph | Me | 1 |
| 1-9 | Bz | Me | 1 |
| 1-10 | 4-Me-Bz | Me | 1 |
| 1-11 | 4-MeO-Bz | Me | 1 |
| 1-12 | 4-F-Bz | Me | 1 |
| 1-13 | 4-Cl-Bz | Me | 1 |
| 1-14 | $CH_2CH_2Ph$ | Me | 1 |
| 1-15 | H | Et | 1 |
| 1-16 | Me | Et | 1 |
| 1-17 | Ph | Et | 1 |
| 1-18 | 4-Me-Ph | Et | 1 |
| 1-19 | 4-MeO-Ph | Et | 1 |
| 1-20 | 4-F-Ph | Et | 1 |
| 1-21 | 4-Cl-Ph | Et | 1 |
| 1-22 | Bz | Et | 1 |
| 1-23 | 4-Me-Bz | Et | 1 |
| 1-24 | 4-MeO-Bz | Et | 1 |
| 1-25 | 4-F-Bz | Et | 1 |
| 1-26 | 4-Cl-Bz | Et | 1 |
| 1-27 | H | Pr | 1 |
| 1-28 | Me | Pr | 1 |
| 1-29 | Ph | Pr | 1 |
| 1-30 | 4-Me-Ph | Pr | 1 |
| 1-31 | 4-MeO-Ph | Pr | 1 |
| 1-32 | 4-F-Ph | Pr | 1 |
| 1-33 | 4-Cl-Ph | Pr | 1 |
| 1-34 | Bz | Pr | 1 |
| 1-35 | 4-Me-Bz | Pr | 1 |
| 1-36 | 4-MeO-Bz | Pr | 1 |
| 1-37 | 4-F-Bz | Pr | 1 |
| 1-38 | 4-Cl-Bz | Pr | 1 |
| 1-39 | H | Me | 2 |
| 1-40 | Me | Me | 2 |
| 1-41 | Ph | Me | 2 |
| 1-42 | 4-Me-Ph | Me | 2 |
| 1-43 | 4-MeO-Ph | Me | 2 |
| 1-44 | 4-F-Ph | Me | 2 |
| 1-45 | 4-Cl-Ph | Me | 2 |
| 1-46 | Bz | Me | 2 |
| 1-47 | 4-Me-Bz | Me | 2 |
| 1-48 | 4-MeO-Bz | Me | 2 |
| 1-49 | 4-F-Bz | Me | 2 |

[Table 1] (continued)

| Compound No. | $R^1$ | $R^2$ | n |
|---|---|---|---|
| 1-50 | 4-Cl-Bz | Me | 2 |
| 1-51 | H | Et | 2 |
| 1-52 | Me | Et | 2 |
| 1-53 | Ph | Et | 2 |
| 1-54 | 4-Me-Ph | Et | 2 |
| 1-55 | 4-MeO-Ph | Et | 2 |
| 1-56 | Bz | Et | 2 |
| 1-57 | 4-Me-Bz | Et | 2 |
| 1-58 | 4-MeO-Bz | Et | 2 |
| 1-59 | H | $Pr^i$ | 1 |
| 1-60 | Me | $Pr^i$ | 1 |
| 1-61 | Ph | $Pr^i$ | 1 |
| 1-62 | 4-Me-Ph | $Pr^i$ | 1 |
| 1-63 | 4-MeO-Ph | $Pr^i$ | 1 |
| 1-64 | 4-F-Ph | $Pr^i$ | 1 |
| 1-65 | 4-Cl-Ph | $Pr^i$ | 1 |
| 1-66 | Bz | $Pr^i$ | 1 |
| 1-67 | 4-Me-Bz | $Pr^i$ | 1 |
| 1-68 | 4-MeO-Bz | $Pr^i$ | 1 |
| 1-69 | 4-F-Bz | $Pr^i$ | 1 |
| 1-70 | 4-Cl-Bz | $Pr^i$ | 1 |
| 1-71 | H | Bu | 1 |
| 1-72 | Me | Bu | 1 |
| 1-73 | Ph | Bu | 1 |
| 1-74 | 4-Me-Ph | Bu | 1 |
| 1-75 | 4-MeO-Ph | Bu | 1 |
| 1-76 | 4-F-Ph | Bu | 1 |
| 1-77 | 4-Cl-Ph | Bu | 1 |
| 1-78 | Bz | Bu | 1 |
| 1-79 | 4-Me-Bz | Bu | 1 |
| 1-80 | 4-MeO-Bz | Bu | 1 |
| 1-81 | 4-F-Bz | Bu | 1 |
| 1-82 | 4-Cl-Bz | Bu | 1 |
| 1-83 | H | $Bu^i$ | 1 |
| 1-84 | Me | $Bu^i$ | 1 |
| 1-85 | Ph | $Bu^i$ | 1 |
| 1-86 | 4-Me-Ph | $Bu^i$ | 1 |
| 1-87 | 4-MeO-Ph | $Bu^i$ | 1 |
| 1-88 | 4-F-Ph | $Bu^i$ | 1 |
| 1-89 | 4-Cl-Ph | $Bu^i$ | 1 |
| 1-90 | Bz | $Bu^i$ | 1 |
| 1-91 | 4-Me-Bz | $Bu^i$ | 1 |
| 1-92 | 4-MeO-Bz | $Bu^i$ | 1 |
| 1-93 | 4-F-Bz | $Bu^i$ | 1 |
| 1-94 | 4-Cl-Bz | $Bu^i$ | 1 |
| 1-95 | H | $Bu^s$ | 1 |
| 1-96 | Me | $Bu^s$ | 1 |
| 1-97 | Ph | $Bu^s$ | 1 |
| 1-98 | 4-Me-Ph | $Bu^s$ | 1 |
| 1-99 | 4-MeO-Ph | $Bu^s$ | 1 |

[Table 1] (continued)

| Compound No. | $R^1$ | $R^2$ | n |
|---|---|---|---|
| 1-100 | Bz | $Bu^s$ | 1 |
| 1-101 | 4-Me-Bz | $Bu^s$ | 1 |
| 1-102 | 4-MeO-Bz | $Bu^s$ | 1 |
| 1-103 | H | $Bu^t$ | 1 |
| 1-104 | Me | But | 1 |
| 1-105 | Ph | $Bu^t$ | 1 |
| 1-106 | 4-Me-Ph | $Bu^t$ | 1 |
| 1-107 | 4-MeO-Ph | $Bu^t$ | 1 |
| 1-108 | Bz | $Bu^t$ | 1 |
| 1-109 | 4-Me-Bz | $Bu^t$ | 1 |
| 1-110 | 4-MeO-Bz | $Bu^t$ | 1 |
| 1-111 | H | Pn | 1 |
| 1-112 | Me | Pn | 1 |
| 1-113 | Ph | Pn | 1 |
| 1-114 | 4-Me-Ph | Pn | 1 |
| 1-115 | 4-MeO-Ph | Pn | 1 |
| 1-116 | Bz | Pn | 1 |
| 1-117 | 4-Me-Bz | Pn | 1 |
| 1-118 | 4-MeO-Bz | Pn | 1 |
| 1-119 | H | Hx | 1 |
| 1-120 | Me | Hx | 1 |
| 1-121 | Ph | Hx | 1 |
| 1-122 | 4-Me-Ph | Hx | 1 |
| 1-123 | 4-MeO-Ph | Hx | 1 |
| 1-124 | Bz | Hx | 1 |
| 1-125 | 4-Me-Bz | Hx | 1 |
| 1-126 | 4-MeO-Bz | Hx | 1 |
| 1-127 | H | Pr | 2 |
| 1-128 | Me | Pr | 2 |
| 1-129 | Ph | Pr | 2 |
| 1-130 | 4-Me-Ph | Pr | 2 |
| 1-131 | 4-MeO-Ph | Pr | 2 |
| 1-132 | Bz | Pr | 2 |
| 1-133 | 4-Me-Bz | Pr | 2 |
| 1-134 | 4-MeO-Bz | Pr | 2 |
| 1-135 | H | $Pr^i$ | 2 |
| 1-136 | Me | $Pr^i$ | 2 |
| 1-137 | Ph | $Pr^i$ | 2 |
| 1-138 | 4-Me-Ph | $Pr^i$ | 2 |
| 1-139 | 4-MeO-Ph | $Pr^i$ | 2 |
| 1-140 | Bz | $Pr^i$ | 2 |
| 1-141 | 4-Me-Bz | $Pr^i$ | 2 |
| 1-142 | 4-MeO-Bz | $Pr^i$ | 2 |
| 1-143 | H | Bu | 2 |
| 1-144 | Me | Bu | 2 |
| 1-145 | Ph | Bu | 2 |
| 1-146 | 4-Me-Ph | Bu | 2 |
| 1-147 | 4-MeO-Ph | Bu | 2 |
| 1-148 | Bz | Bu | 2 |
| 1-149 | 4-Me-Bz | Bu | 2 |

[Table 1] (continued)

| Compound No. | $R^1$ | $R^2$ | n |
|---|---|---|---|
| 1-150 | 4-MeO-Bz | Bu | 2 |
| 1-151 | H | $Bu^i$ | 2 |
| 1-152 | Me | $Bu^i$ | 2 |
| 1-153 | Ph | $Bu^i$ | 2 |
| 1-154 | 4-Me-Ph | $Bu^i$ | 2 |
| 1-155 | 4-MeO-Ph | $Bu^i$ | 2 |
| 1-156 | Bz | $Bu^i$ | 2 |
| 1-157 | 4-Me-Bz | $Bu^i$ | 2 |
| 1-158 | 4-MeO-Bz | $Bu^i$ | 2 |

In the above Table 1, the abbreviation indicates the following group.

Bz ... Benzyl
Bu ... Butyl
$Bu^i$ ... Isobutyl
$Bu^s$ ... s-Butyl
$Bu^t$ ... t-Butyl
Et ... Ethyl
Hx ... Hexyl
Me ... Methyl
Ph ... Phenyl
Pn ... Pentyl
Pr ... Propyl
$Pr^i$ ... Isopropyl

In the above Table 1, preferred are compounds of Compound Nos. 1-1, 1-2, 1-3, 1-6, 1-7, 1-9, 1-10, 1-11, 1-12, 1-13, 1-14, 1-15, 1-27, 1-28, 1-29, 1-30, 1-31, 1-32, 1-33, 1-34, 1-36, 1-39, 1-40, 1-43, 1-59, 1-60, 1-71, 1-72, 1-73, 1-74, 1-75, 1-78, 1-80, 1-83, 1-84, 1-85, 1-87, 1-90, 1-92, 1-95, 1-103, 1-111, 1-112, 1-116, 1-119, 1-120, 1-123, 1-124, 1-127, 1-143 and 1-151;

more preferred are compounds of Compounds Nos. 1-1, 1-2, 1-3, 1-6, 1-9, 1-10, 1-12, 1-14, 1-27, 1-28, 1-29, 1-31, 3-32, 1-33, 1-34, 1-36, 1-60, 1-71, 1-72, 1-73, 1-75, 1-78, 1-80, 1-83, 1-84, 1-85, 1-87, 1-90, 1-92, 1-95, 1-103, 1-111, 1-112, 1-116, 1-119, 1-120, 1-123, 1-124, 1-127, 1-143 and 1-151;
and particularly preferred are the compounds of

Compound No. 1-1: (4R)-N-[(1S)-1-methyl-2-nitroxyethyl]-2-oxothiazolidin-4-yl-carboxamide,
Compound No. 1-2: (4R)-N-[(1S)-1-methyl-2-nitroxyethyl]-5-methyl-2-oxothiazolidin-4-yl-carboxamide,
Compound No. 1-6: (4R)-N-[(1S)-1-methyl-2-nitroxyethyl]-5-(4-methoxyphenyl)-2-oxothiazolidin-4-yl-carboxamide,
Compound No. 1-9: (4R)-N-[(1S)-1-methyl-2-nitroxyethyl]-5-benzyl-2-oxothiazolidin-4-yl-carboxamide,
Compound No. 1-15: (4R)-N-[(1S)-1-ethyl-2-nitroxyethyl]-2-oxothiazolidin-4-yl-carboxamide,
Compound No. 1-27: (4R)-N-[(1S)-1-propyl-2-nitroxyethyl]-2-oxothiazolidin-4-yl-carboxamide,
Compound No. 1-28: (4R)-N-[(1S)-1-propyl-2-nitroxyethyll-5-methyl-2-oxothiazolidin-4-yl-carboxamide,
Compound No. 1-31: (4R)-N-[(1S)-1-propyl-2-nitroxyethyl] -5- (4-methoxyphenyl)-2-oxothiazolidin-4-yl-carboxamide,
Compound No. 1-34: (4R)-N-[(1S)-1-propyl-2-nitroxyethyl] -5-benzyl-2-oxothiazolidin-4-yl-carboxamide,
Compound No. 1-71: (4R)-N-[(lS)-1-butyl-2-nitroxyethyl]-2-oxothiazolidin-4-yl-carboxamide,
Compound No. 1-72: (4R)-N-[(1S)-1-butyl-2-nitroxyethyl]-S-methyl-2-oxothiazolidin-4-yl-carboxamide,
Compound No. 1-75: (4R)-N-[(1S)-1-butyl-2-nitroxyethyl]-5-(4-methoxyphenyl)-2-oxothiazolidin-4-yl-carboxamide,
Compound No. 1-78: (4R)-N-[(1S)-1-butyl-2-nitroxyethyl]-5-benzyl-2-oxothiazolidin-4-yl-carboxamide,
Compound No. 1-83: (4R)-N-[(1S)-1-isobutyl-2-nitroxyethyl]-2-oxothiazolidin-4-yl-carboxamide,
Compound No. 1-84: (4R)-N-[(1S) -1-isobutyl-2-nitroxyethyl]-5-methyl-2-oxothiazolidin-4-yl-carboxamide,
Compound No. 1-87: (4R)-N-[(1S)-1-isobutyl-2-nitroxyethyl]-5-(4-methoxyphenyl)-2-oxothiazolidin-4-yl-carboxamide and

Compound No. 1-90: (4R)-N-[(1S)-1-isobutyl-2-nitroxyethyl] -5-benzyl-2-oxothiazolidin-4-yl-carboxamide

The preferred compounds of the general formula (II) can be specifically exemplified in Table 2 and Table 3. The compounds of Table 2 and Table 3 have the structural formulae of (II-1) and (II-2), respectively.

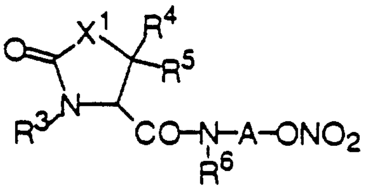

(II-1)

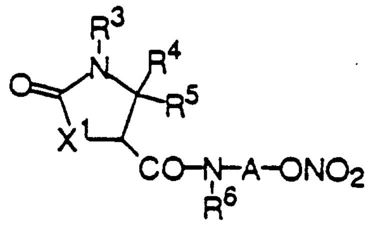

(II-2)

[Table 2]

| Compound No. | $R^3$ | $R^4$ | $R^5$ | $R^6$ | A | $X^1$ |
|---|---|---|---|---|---|---|
| 2-1 | H | H | H | H | $(CH_2)_2$ | S |
| 2-2 | Me | H | H | H | $(CH_2)_2$ | S |
| 2-3 | Et | H | H | H | $(CH_2)_2$ | S |
| 2-4 | $PhCH_2$ | H | H | H | $(CH_2)_2$ | S |
| 2-5 | H | Me | H | H | $(CH_2)_2$ | S |
| 2-6 | H | Et | H | H | $(CH_2)_2$ | S |
| 2-7 | H | Ph | H | H | $(CH_2)_2$ | S |
| 2-8 | H | 2-Thi | H | H | $(CH_2)_2$ | S |
| 2-9 | H | 3-Thi | H | H | $(CH_2)_2$ | S |
| 2-10 | H | 2-Fur | H | H | $(CH_2)_2$ | S |
| 2-11 | H | 3-Fur | H | H | $(CH_2)_2$ | S |
| 2-12 | H | $3-NO_2-Ph$ | H | H | $(CH_2)_2$ | S |
| 2-13 | H | 4-Cl-Ph | H | H | $(CH_2)_2$ | S |
| 2-14 | H | 4-MeO-Ph | H | H | $(CH_2)_2$ | S |
| 2-15 | H | 4-Thiz | H | H | $(CH_2)_2$ | S |
| 2-16 | H | 3-Pyr | H | H | $(CH_2)_2$ | S |
| 2-17 | H | Me | Me | H | $(CH_2)_2$ | S |
| 2-18 | Me | Me | Me | H | $(CH_2)_2$ | S |
| 2-19 | Me | Me | Me | Me | $(CH_2)_2$ | S |
| 2-20 | Et | Ph | H | H | $(CH_2)_3$ | S |
| 2-21 | Et | Et | H | Me | $(CH_2)_4$ | S |
| 2-22 | $PhCH_2$ | Me | H | Et | $(CH_2)_2$ | S |
| 2-23 | $PhCH_2$ | Ph | H | Pr | $(CH_2)_4$ | S |
| 2-24 | Bu | H | H | H | $(CH_2)_2$ | S |
| 2-25 | H | 1-Naph | H | H | $(CH_2)_2$ | S |
| 2-26 | H | H | H | Me | $(CH_2)_2$ | S |
| 2-27 | H | H | H | $PhCH_2$ | $(CH_2)_2$ | S |

[Table 2] (continued)

| Compound No. | $R^3$ | $R^4$ | $R^5$ | $R^6$ | A | $X^1$ |
|---|---|---|---|---|---|---|
| 2-28 | H | $PhCH_2$ | H | H | $(CH_2)_2$ | S |
| 2-29 | $PhCH_2$ | H | H | H | $(CH_2)_3$ | S |
| 2-30 | H | H | H | H | $CH(Me)CH_2$ | S |
| 2-31 | H | H | H | H | $CH_2CH(Me)$ | S |
| 2-32 | H | H | H | H | $(CH_2)_5$ | S |
| 2-33 | H | H | H | H | $(CH_2)_6$ | S |
| 2-34 | H | H | H | H | $(CH_2)_2$ | O |
| 2-35 | Me | H | H | H | $(CH_2)_2$ | O |
| 2-36 | Et | H | H | H | $(CH_2)_2$ | O |
| 2-37 | $PhCH_2$ | H | H | H | $(CH_2)_2$ | O |
| 2-38 | H | Me | H. | H | $(CH_2)_2$ | O |
| 2-39 | H | Et | H | H | $(CH_2)_2$ | O |
| 2-40 | H | Ph | H | H | $(CH_2)_2$ | O |
| 2-41 | H | 2-Thi | H | H | $(CH_2)_2$ | O |
| 2-42 | H | 3-Thi | H | H | $(CH_2)_2$ | O |
| 2-43 | H | 2-Fur | H | H | $(CH_2)_2$ | O |
| 2-44 | H | 3-Fur | H | H | $(CH_2)_2$ | O |
| 2-45 | H | 3-$NO_2$-Ph | H | H | $(CH_2)_2$ | O |
| 2-46 | H | 4-Cl-Ph | H | H | $(CH_2)_2$ | O |
| 2-47 | H | 4-MeO-Ph | H | H | $(CH_2)_2$ | O |
| 2-48 | H | 4-Thiz | H | H | $(CH_2)_2$ | O |
| 2-49 | H | 3-Pyr | H | H | $(CH_2)_2$ | O |
| 2-50 | H | Me | Me | H | $(CH_2)_2$ | O |
| 2-51 | Me | Me | Me | H | $(CH_2)_2$ | O |
| 2-52 | Me | Me | Me | Me | $(CH_2)_2$ | O |
| 2-53 | Et | Ph | H | H | $(CH_2)_3$ | O |
| 2-54 | Et | Et | H | Me | $(CH_2)_4$ | O |
| 2-55 | $PhCH_2$ | Me | H | Et | $(CH_2)_2$ | O |
| 2-56 | $PhCH_2$ | Ph | H | Pr | $(CH_2)_4$ | 0 |
| 2-57 | Bu | H | H | H | $(CH_2)_2$ | O |
| 2-58 | H | 1-Naph | H | H | $(CH_2)_2$ | O |
| 2-59 | H | H | H | Me | $(CH_2)_2$ | O |
| 2-60 | H | H | H | $PhCH_2$ | $(CH_2)_2$ | O |
| 2-61 | H | $PhCH_2$ | H | H | $(CH_2)_2$ | O |
| 2-62 | H | H | H | H | $(CH_2)_3$ | O |
| 2-63 | H | H | H | H | $CH(Me)CH_2$ | O |
| 2-64 | H | H | H | H | $CH_2CH(Me)$ | O |
| 2-65 | H | H | H | H | $(CH_2)_5$ | O |
| 2-66 | H | H | H | H | $(CH_2)_6$ | O |
| 2-67 | H | H | H | H | $(CH_2)_4$ | S |
| 2-68 | H | H | H | H | $(CH_2)_3$ | S |
| 2-69 | H | 4-Me-$CH_2$Ph | H | H | $(CH_2)_2$ | S |
| 2-70 | H | 4-MeO-$CH_2$Ph | H | H | $(CH_2)_2$ | S |
| 2-71 | H | 4-F-$CH_2$Ph | H | H | $(CH_2)_2$ | S |
| 2-72 | H | 4-Cl-$CH_2$Ph | H | H | $(CH_2)_2$ | S |
| 2-73 | H | 4-OH-$CH_2$Ph | H | H | $(CH_2)_2$ | S |
| 2-74 | H | 4-Me-Ph | H | H | $(CH_2)_2$ | S |
| 2-75 | H | 4-F-Ph | H | H | $(CH_2)_2$ | S |
| 2-76 | H | 4-OH-Ph | H | H | $(CH_2)_2$ | S |
| 2-77 | H | 4-Me-$CH_2$Ph | H | H | $(CH_2)_2$ | O |

[Table 2] (continued)

| Compound No. | $R^3$ | $R^4$ | $R^5$ | $R^6$ | A | $X^1$ |
|---|---|---|---|---|---|---|
| 2-78 | H | 4-MeO-$CH_2$Ph | H | H | $(CH_2)_2$ | O |
| 2-79 | H | 4-F-$CH_2$Ph | H | H | $(CH_2)_2$ | O |
| 2-80 | H | 4-Cl-$CH_2$Ph | H | H | $(CH_2)_2$ | O |
| 2-81 | H | 4-OH-$CH_2$Ph | H | H | $(CH_2)_2$ | O |
| 2-82 | H | 4-Me-Ph | H | H | $(CH_2)_2$ | O |
| 2-83 | H | 4-F-Ph | H | H | $(CH_2)_2$ | O |
| 2-84 | H | 4-OH-Ph | H | H | $(CH_2)_2$ | O |
| 2-85 | H | H | H | H | $(CH_2)_4$ | O |
| 2-86 | H | Me | H | H | CH(Me)$CH_2$ | S |
| 2-87 | H | 3-Fur | H | H | CH(Me)$CH_2$ | S |
| 2-88 | H | 4-MeO-Ph | H | H | CH(Me)$CH_2$ | S |
| 2-89 | H | Ph$CH_2$ | H | H | CH(Me)$CH_2$ | S |
| 2-90 | H | Me | H | H | CH(Me)$CH_2$ | O |
| 2-91 | H | 3-Fur | H | H | CH(Me)$CH_2$ | O |
| 2-92 | H | 4-MeO-Ph | H | H | CH(Me)$CH_2$ | O |
| 2-93 | H | Ph$CH_2$ | H | H | CH(Me)$CH_2$ | O |

[Table 3]

| Compound No. | $R^3$ | $R^4$ | $R^5$ | $R^6$ | A | $X^1$ |
|---|---|---|---|---|---|---|
| 3-1 | H | H | H | H | $(CH_2)_2$ | S |
| 3-2 | Me | H | H | H | $(CH_2)_2$ | S |
| 3-3 | Et | H | H | H | $(CH_2)_2$ | S |
| 3-4 | Ph$CH_2$ | H | H | H | $(CH_2)_2$ | S |
| 3-5 | H | Me | H | H | $(CH_2)_2$ | S |
| 3-6 | H | Et | H | H | $(CH_2)_2$ | S |
| 3-7 | H | Ph | H | H | $(CH_2)_2$ | S |
| 3-8 | H | 2-Thi | H | H | $(CH_2)_2$ | S |
| 3-9 | H | 3-Thi | H | H | $(CH_2)_2$ | S |
| 3-10 | H | 2-Fur | H | H | $(CH_2)_2$ | S |
| 3-11 | H | 3-Fur | H | H | $(CH_2)_2$ | S |
| 3-12 | H | 3-$NO_2$-Ph | H | H | $(CH_2)_2$ | S |
| 3-13 | H | 4-Cl-Ph | H | H | $(CH_2)_2$ | S |
| 3-14 | H | 4-MeO-Ph | H | H | $(CH_2)_2$ | S |
| 3-15 | H | 4-Thiz | H | H | $(CH_2)_2$ | S |
| 3-16 | H | 3-Pyr | H | H | $(CH_2)_2$ | S |
| 3-17 | H | Me | Me | H | $(CH_2)_2$ | S |
| 3-18 | Me | Me | Me | H | $(CH_2)_2$ | S |
| 3-19 | Me | Me | Me | Me | $(CH_2)_2$ | S |
| 3-20 | Et | Ph | H | H | $(CH_2)_2$ | S |
| 3-21 | Et | Et | H | Me | $(CH_2)_4$ | S |
| 3-22 | Ph$CH_2$ | Me | H | Et | $(CH_2)_2$ | S |
| 3-23 | Ph$CH_2$ | Ph | H | Pr | $(CH_2)_4$ | S |
| 3-24 | Bu | H | H | H | $(CH_2)_2$ | S |
| 3-25 | H | 1-Naph | H | H | $(CH_2)_2$ | S |
| 3-26 | H | H | H | Me | $(CH_2)_2$ | S |
| 3-27 | H | H | H | Ph$CH_2$ | $(CH_2)_2$ | S |
| 3-28 | H | Ph$CH_2$ | H | H | $(CH_2)_2$ | S |
| 3-29 | H | H | H | H | $(CH_2)_3$ | S |

[Table 3] (continued)

| Compound No. | $R^3$ | $R^4$ | $R^5$ | $R^6$ | A | $X^1$ |
|---|---|---|---|---|---|---|
| 3-30 | H | H | H | H | $CH(Me)CH_2$ | S |
| 3-31 | H | H | H | H | $CH_2CH(Me)$ | S |
| 3-32 | H | H | H | H | $(CH_2)_5$ | S |
| 3-33 | H | H | H | H | $(CH_2)_6$ | S |
| 3-34 | H | H | H | H | $(CH_2)_2$ | O |
| 3-35 | Me | H | H | H | $(CH_2)_2$ | O |
| 3-36 | Et | H | H | H | $(CH_2)_2$ | O |
| 3-37 | $PhCH_2$ | H | H | H | $(CH_2)_2$ | O |
| 3-38 | H | Me | H | H | $(CH_2)_2$ | O |
| 3-39 | H | Et | H | H | $(CH_2)_2$ | O |
| 3-40 | H | Ph | H | H | $(CH_2)_2$ | O |
| 3-41 | H | 2-Thi | H | H | $(CH_2)_2$ | O |
| 3-42 | H | 3-Thi | H | H | $(CH_2)_2$ | O |
| 3-43 | H | 2-Fur | H | H | $(CH_2)_2$ | O |
| 3-44 | H | 3-Fur | H | H | $(CH_2)_2$ | O |
| 3-45 | H | $3-NO_2-PH$ | H | H | $(CH_2)_2$ | O |
| 3-46 | H | 4-Cl-Ph | H | H | $(CH_2)_2$ | O |
| 3-47 | H | 4-MeO-Ph | H | H | $(CH_2)_2$ | O |
| 3-48 | H | 4-Thiz | H | H | $(CH_2)_2$ | O |
| 3-49 | H | 3-Pyr | H | H | $(CH_2)_2$ | O |
| 3-50 | H | Me | Me | H | $(CH_2)_2$ | O |
| 3-51 | Me | Me | Me | H | $(CH_2)_2$ | O |
| 3-52 | Me | Me | Me | Me | $(CH_2)_2$ | O |
| 3-53 | Et | Ph | H | H | $(CH_2)_3$ | O |
| 3-54 | Et | Et | H | Me | $(CH_2)_4$ | O |
| 3-55 | $PhCH_2$ | Me | H | Et | $(CH_2)_2$ | O |
| 3-56 | $PhCH_2$ | Ph | H | Pr | $(CH_2)_4$ | O |
| 3-57 | Bu | H | H | H | $(CH_2)_2$ | O |
| 3-58 | H | 1-Naph | H | H | $(CH_2)_2$ | O |
| 3-59 | H | H | H | Me | $(CH_2)_2$ | O |
| 3-60 | H | H | H | $PhCH_2$ | $(CH_2)_2$ | O |
| 3-61 | H | $PhCH_2$ | H | H | $(CH_2)_2$ | O |
| 3-62 | H | H | H | H | $(CH_2)_3$ | O |
| 3-63 | H | H | H | H | $CH(Me)CH_2$ | O |
| 3-64 | H | H | H | H | $CH_2CH(Me)$ | O |
| 3-65 | H | 4-Me-Ph | H | H | $(CH_2)_2$ | O |
| 3-66 | H | 4-Me-Ph | H | H | $(CH_2)_2$ | S |
| 3-67 | H | $4-Me-CH_2Ph$ | H | H | $(CH_2)_2$ | S |
| 3-68 | H | $4-MeO-CH_2Ph$ | H | H | $(CH_2)_2$ | S |
| 3-69 | H | $4-F-CH_2Ph$ | H | H | $(CH_2)_2$ | S |
| 3-70 | H | $4-Cl-CH_2Ph$ | H | H | $(CH_2)_2$ | S |
| 3-71 | H | $4-OH-CH_2Ph$ | H | H | $(CH_2)_2$ | S |
| 3-72 | H | 4-F-Ph | H | H | $(CH_2)_2$ | S |
| 3-73 | H | 4-OH-Ph | H | H | $(CH_2)_2$ | S |
| 3-74 | H | $4-Me-CH_2Ph$ | H | H | $(CH_2)_2$ | O |
| 3-75 | H | $4-MeO-CH_2Ph$ | H | H | $(CH_2)_2$ | O |
| 3-76 | H | $4-F-CH_2Ph$ | H | H | $(CH_2)_2$ | O |
| 3-77 | H | $4-Cl-CH_2Ph$ | H | H | $(CH_2)_2$ | O |
| 3-78 | H | $4-OH-CH_2Ph$ | H | H | $(CH_2)_2$ | O |
| 3-79 | H | 4-F-Ph | H | H | $(CH_2)_2$ | O |

[Table 3] (continued)

| Compound No. | $R^3$ | $R^4$ | $R^5$ | $R^6$ | A | $X^1$ |
|---|---|---|---|---|---|---|
| 3-80 | H | 4-OH-Ph | H | H | $(CH_2)_2$ | O |
| 3-81 | H | H | H | H | $(CH_2)_4$ | S |
| 3-82 | H | H | H | H | $(CH_2)_4$ | O |
| 3-83 | H | Me | H | H | $CH(Me)CH_2$ | S |
| 3-84 | H | 3-Fur | H | H | $CH(Me)CH_2$ | S |
| 3-85 | H | 4-MeO-Ph | H | H | $CH(Me)CH_2$ | S |
| 3-86 | H | $PhCH_2$ | H | H | $CH(Me)CH_2$ | S |
| 3-87 | H | Me | H | H | $CH(Me)CH_2$ | O |
| 3-88 | H | 3-Fur | H | H | $CH(Me)CH_2$ | O |
| 3-89 | H | 4-MeO-Ph | H | H | $CH(Me)CH_2$ | O |
| 3-90 | H | $PhCH_2$ | H | H | $CH(Me)CH_2$ | O |

In the above Tables 2 and 3, the abbreviation indicates the following group.

Bu ... Butyl
Et ... Ethyl
Fur .. Furyl
Me ... Methyl
Naph ... Naphthyl
Ph ... Phenyl
Pr ... Propyl
Prd .. Pyridyl
Thi ... Thienyl Thiz ... Thiazolyl

In the above Tables, preferred are compounds of Compound Nos. 2-1, 2-2, 2-5, 2-7, 2-8, 2-9, 2-10, 2-11, 2-12, 2-13, 2-14, 2-16, 2-17, 2-25, 2-26, 2-28, 2-30, 2-31, 2-34, 2-35, 2-38, 2-40, 2-41, 2-42, 2-43, 2-44, 2-47, 2-61, 2-63, 2-67, 2-68, 2-69, 2-70, 2-71, 2-72, 2-73, 2-74, 2-75, 2-76, 2-77, 2-78, 2-79, 2-80, 2-81, 2-82, 2-83, 2-84, 2-85, 2-86, 2-87, 2-88, 2-89, 2-90, 2-91, 2-92, 2-93, 3-1, 3-5, 3-7, 3-14, 3-30, 3-34, 3-38, 3-40, 3-47, 3-65, 3-66, 3-67, 3-68, 3-69, 3-70, 3-71, 3-72, 3-73, 3-83, 3-84, 3-85, 3-86, 3-87, 3-88, 3-89 and 3-90;

more preferred are compounds of Compound Nos. 2-1, 2-2, 2-5, 2-7, 2-8, 2-14, 2-25, 2-28, 2-30, 2-34, 2-38, 2-41, 2-44, 2-47, 2-61, 2-63, 2-69, 2-70, 2-74, 2-78, 2-86, 2-88, 2-89, 2-90, 2-91, 2-92, 2-93, 3-1, 3-7, 3-14, 3-30, 3-66, 3-67, 3-68 and 3-85;

and particularly preferred are the compounds of

Compound No. 2-1: N-(2-nitroxyethyl)-2-oxothiazolidin-4-ylcarboxamide,
Compound No. 2-5: N-(2-nitroxyethyl)-5-methyl-2-oxothiazolidin-4-ylcarboxamide,
Compound No. 2-14: N-(2-nitroxyethyl)-5-(4-methoxyphenyl)-2-oxothiazolidin-4-ylcarboxamide,
Compound No. 2-28: N-(2-nitroxyethyl)-5-benzyl-2-oxothiazolidin-4-ylcarboxamide,
Compound No. 2-30: N-(1-methyl-2-nitroxyethyl)-2-oxothiazolidin-4-ylcarboxamide,
Compound No. 2-34: N-(2-nitroxyethyl)-2-oxoxazolidin-4-ylcarboxamide,
Compound No. 2-44: N-(2-nitroxyethyl)-5-(3-furyl)-2-oxoxazolidin-4-ylcarboxamide,
Compound No. 2-61: N-(2-nitroxyethyl)-5-benzyl-2-oxoxazolidin-4-ylcarboxamide,
Compound No. 2-63: N-(1-methyl-2-nitroxyethyl)-2-oxoxazolidin-4-ylcarboxamide,
Compound No. 2-86: N-(1-methyl-2-nitroxyethyl)-5-methyl-2-oxothiazolidin-4-ylcarboxamide,
Compound No. 2-88: N-(1-methyl-2-nitroxyethyl )-5-(4-methoxyphenyl)-2-oxothiazolidin-4-ylcarboxamide,
Compound No. 2-89: N-(1-methyl-2-nitroxyethyl)-5-benzyl-2-oxothiazolidin-4-ylcarboxamide,
Compound No. 2-91: N-(1-methyl-2-nitroxyethyl)-5-(3-furyl)-2-oxoxazolidin-4-ylcarboxamide,
Compound No. 2-93: N-(1-methyl-2-nitroxyethyl)-5-benzyl-2-oxoxazolidin-4-ylcarboxamide,
Compound No. 3-1: N-(2-nitroxyethyl)-2-oxothiazolidin-5-ylcarboxamide,
Compound No. 3-14: N-(2-nitroxyethyl)-4-(4-methoxyphenyl)-2-oxothiazolidin-5-ylcarboxamide,
Compound No. 3-30: N-(1-methyl-2-nitroxyethyl)-2-oxothiazolidin-5-ylcarboxamide and
Compound No. 3-85: N-(1-methyl-2-nitroxyethyl)-4-(4-methoxyphenyl)-2-oxothiazolidin-5-ylcarboxamide.

The compound of the present invention having the general formula (I) is easily prepared according to the following methods.

Method A

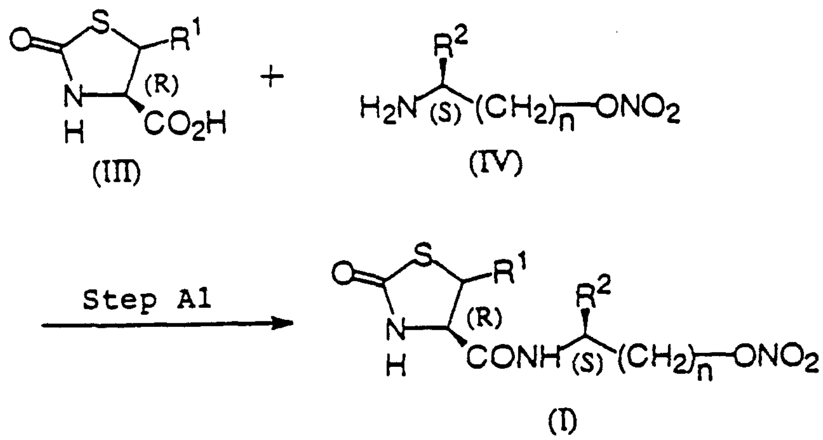


(III) (IV) (I)

Method B

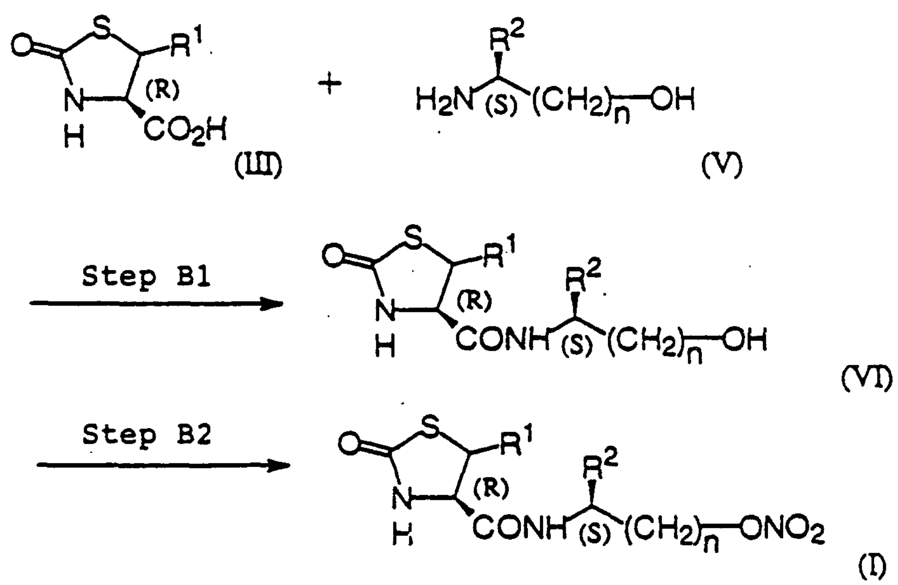


(III) (V) (VI) (I)

Method C

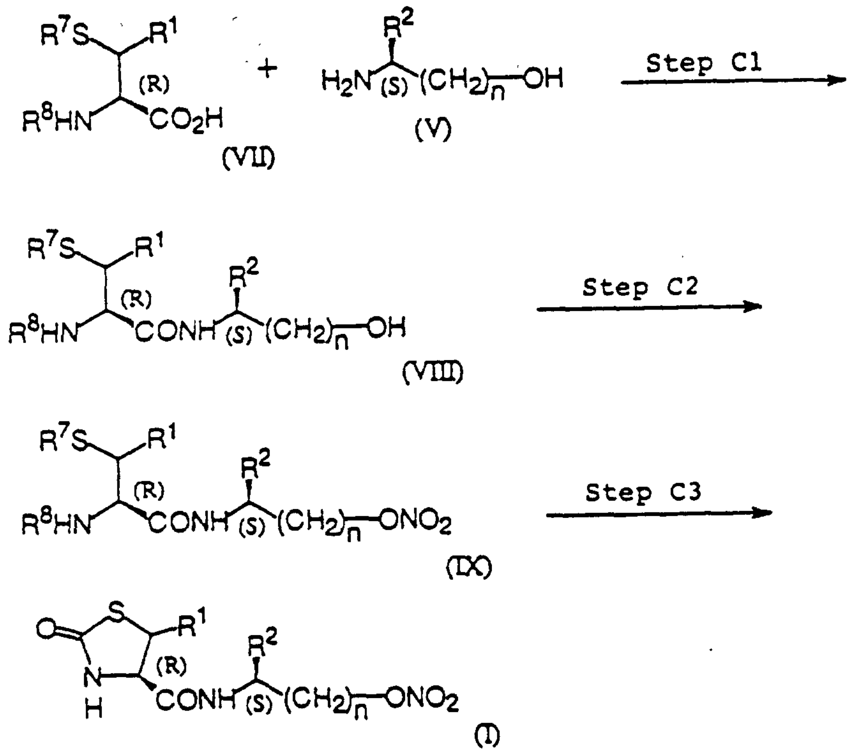


(VII) (V) (VIII) (IX) (I)

In the above formulae, $R^1$, $R^2$ and n have the same meanings as defined above, $R^7$ represents a protective group of a mercapto group and $R^8$ represents a protective group of an amino group.

The protective group of the mercapto group is not particularly limited so long as it is well known in the field of synthetic organic chemistry and includes preferably a tri-substituted silyl group having a substituent selected from the group consisting of a $C_1$-$C_4$ alkyl group, a phenyl group and a phenyl group substituted with $C_1$-$C_4$ alkyl, $C_1$-$C_4$ alkoxy or halogen, a benzyl group, a benzyl group substituted with $C_1$-$C_4$ alkyl, $C_1$-$C_4$ alkoxy or halogen, a benzyloxycarbonyl group, a benzyloxycarbonyl group substituted with $C_1$-$C_4$ alkyl, $C_1$-$C_4$ alkoxy or halogen, a t-butyl group or a t-butoxycarbonyl group, more preferably a trimethylsilyl group, a triethylsilyl group, a t-butyldimethylsilyl group, a phenyldimethylsilyl group, a methoxybenzyl group, a dimethoxybenzyl group, a methoxybenzyloxycarbonyl group, a dimethoxybenzyloxycarbonyl group or a t-butoxycarbonyl group, still more preferably a t-butyldimethylsilyl group, a 4-methoxybenzyl group, a 4-methoxybenzyloxycarbonyl group or a t-butoxycarbonyl group, and particularly preferably a t-butoxycarbonyl group.

The protective group of the amino group is not particularly limited so long as it is well known in the field of synthetic organic chemistry and includes preferably a tri-substituted silyl group having a substituent selected from the group consisting of a $C_1$-$C_4$ alkyl group, a phenyl group and a phenyl group substituted with $C_1$-$C_4$ alkyl, $C_1$-$C_4$ alkoxy or halogen, a benzyl group, a benzyl group substituted with $C_1$-$C_4$ alkyl, $C_1$-$C_4$ alkoxy or halogen, a benzyloxycarbonyl group, a benzyloxycarbonyl group substituted with $C_1$-$C_4$ alkyl, $C_1$-$C_4$ alkoxy or halogen, a t-butyl group or a t-butoxycarbonyl group, more preferably a trimethylsilyl group, a triethylsilyl group, a t-butyldimethylsilyl group, a phenyldimethylsilyl group, a methoxybenzyl group, a dimethoxybenzyl group, a methoxybenzyloxycarbonyl group, a dimethoxybenzyloxycarbonyl group or a t-butoxycarbonyl group, still more preferably a t-butyldimethylsilyl group, a 4-methoxybenzyl group, a 4-methoxybenzyloxycarbonyl group or a t-butoxycarbonyl group, and particularly preferably a t-butoxycarbonyl group.

Method A is a method for preparing the compound (I).

Step Al is to prepare a compound having the general formula (I) and is carried out by reacting a compound having the general formula (III) or a reactive derivative thereof (acid halides, mixed acid anhydrides or active esters) with a compound having the general formula (IV) or its acid addition salt (for example, mineral acid salts such as hydrochlorides, nitrates and sulfates) in an inert solvent, and is carried out, for example, by an acid halide method, a mixed acid anhydride method, an active ester method or a condensation method.

The acid halide method is carried out by reacting the compound (III) with a halogenating agent (for example, thionyl chloride, oxalyl chloride, phosphorus pentachloride, etc.) in an inert solvent to prepare the acid halide, and by

reacting the acid halide with the compound (IV) or an acid addition salt thereof in an inert solvent in the presence or absence of a base.

The base employable here may include, for example, organic amines such as triethylamine, N-methylmorpholine, pyridine and 4-dimethylaminopyridine; alkali metal hydrogencarbonates such as sodium hydrogencarbonate and potassium hydrogencarbonate; and alkali metal carbonates such as sodium carbonate and potassium carbonate, and preferably organic amines.

The inert solvent employable here is not particularly limited so long as it does not affect the reaction and may include, for example, hydrocarbons such as hexane, cyclohexane, benzene, toluene and xylene; halogenated hydrocarbons such as dichloromethane, 1,2-dichloroethane and carbon tetrachloride; ethers such as ether, tetrahydrofuran and dioxane; ketones such as acetone; amides such as N,N-dimethylformamide, N,N-dimethylacetamide, N-methyl-2-pyrrolidone and hexamethylphosphoramide; and sulfoxides such as dimethyl sulfoxide, and preferably hydrocarbons, halogenated hydrocarbons, ethers or amides.

The reaction temperature varies depending on the starting compounds (III) and (IV), the kind of solvent etc., and the reaction temperatures for both the reaction of the halogenating agent with the compound (III) and the reaction of the acid halide with the compound (IV) are usually -20°C to 150°C. Preferably, the reaction temperature for the former reaction is -10°C to 50°C, and for the latter reaction is 0°C to 100°C. The reaction time varies depending on the reaction temperature etc., and the reaction time of both reactions is usually 15 minutes to 24 hours (preferably 30 minutes to 16 hours).

The mixed acid anhydride method is carried out by reacting a $C_1$-$C_6$ alkyl halogenocarbonate, a di-$C_1$-$C_6$ alkylcyanophosphoric acid or a di-$C_6$-$C_{10}$ arylphosphoryl azide with the compound (III) to prepare the mixed acid anhydride and by reacting the resulting mixed acid anhydride with the compound (IV) or its acid addition salt.

The reaction for preparing the mixed acid anhydride is carried out by reacting a $C_1$-$C_6$ alkyl halogenocarbonate such as methyl chlorocarbonate, ethyl chlorocarbonate, isobutyl chlorocarbonate and hexyl chlorocarbonate (preferably ethyl chlorocarbonate or isobutyl chlorocarbonate), a di-$C_1$-$C_6$ alkylcyanophosphoric acid such as dimethylcyanophosphoric acid, diethylcyanophosphoric acid and dihexylcyanophosphoric acid (preferably diethylcyanophosphoric acid) or a di-$C_6$-$C_{10}$ arylphosphoryl azide such as diphenylphosphoryl azide, di(p-nitrophenyl)phosphoryl azide and dinaphthylphosphoryl azide (preferably diphenylphosphoryl azide) with the compound (III), preferably in an inert solvent in the presence of a base.

The base and the inert solvent employable here are similar to those employable in the acid halide method.

The reaction temperature varies depending on the starting compound (III), the kind of solvent, etc., and is usually -20°C to 50°C (preferably 0°C to 30°C). The reaction time varies depending on the reaction temperature etc. and is usually 15 minutes to 24 hours (preferably 30 minutes to 16 hours).

The reaction of the mixed acid anhydride with the compound (IV) or its acid addition salt is preferably carried out in an inert solvent in the presence or absence of a base. The base and the inert solvent employable here are similar to those employable in the acid halide method.

The reaction temperature varies depending on the starting compound (IV), the kind of solvent, etc., and is usually -20°C to 100°C (preferably -10°C to 50°C). The reaction time varies depending on the reaction temperature etc., and is usually 15 minutes to 24 hours (preferably 30 minutes to 16 hours).

In the case where dialkylcyanophosphoric acid or diarylphosphoryl azide is used, the present method can be carried out by reacting the compound (III) with the compound (IV) directly in the presence of a base.

The active ester method can be carried out by reacting the compound (III) with an active esterifying agent (for example, an N-hydroxy compound such as N-hydroxysuccinimide or N-hydroxybenzotriazole in the presence of a condensation agent (for example, dicyclohexylcarbodiimide, carbonyldiimidazole) to prepare the active ester, and by reacting the active ester with the compound (IV) or its acid addition salt.

The reaction for preparing the active ester is preferably carried out in an inert solvent, and the inert solvent employable here is similar to that employable in the acid halide method.

The reaction temperature varies depending on the starting compounds (III) and (IV), the kind of solvent, etc., the reaction temperature for the active esterification reaction is usually -20°C to 50°C (preferably -10°C to 30°C), and the reaction temperature for the reaction of the active ester compound with the compound (IV) is usually -20°C to 50°C (preferably -10°C to 30°C). The reaction time varies depending on the reaction temperature etc., and the reaction times for both reactions are usually 15 minutes to 24 hours (preferably 30 minutes to 16 hours).

The condensation method can be carried out by reacting, the compound (III) with the compound (IV) or an acid addition salt thereof directly in the presence of a condensation agent (for example, dicyclohexylcarbodiimide, carbonyldiimidazole, 1-(N,N-dimethylaminopropyl)-3-ethylcarbodiimide hydrochloride and the like). The present reaction is carried out in the similar manner to the reaction for preparing the active ester.

After completion of the reaction, the desired compound in each reaction is collected from the reaction mixture by conventional procedures. For example, the desired compound of each reaction can be obtained by appropriately separating the insolubles by filtration and collecting the precipitated crystal by filtration; or by appropriately separating

the insolubles by filtration, appropriately neutralizing, distilling off the solvent, adding water to the reaction mixture, extracting the mixture with a water-immiscible organic solvent such as ethyl acetate, drying the organic layer and evaporating the extracting solvent. If necessary, the compound thus obtained can be further purified by conventional procedures, for example, recrystallization, column chromatography and the like .

The starting compound (III) of Method A is known or is easily prepared according to known methods or methods which are similar thereto [for example, Tetrahedron, 45, 7459 (1989), J. Am. Chem. Soc., 79, 5203 (1957), J. Am. Chem. Soc., 111, 6354 (1989), etc.].

Method B is another method for preparing the compound (I).

Step B1 is to prepare a compound having the general formula (VI) and is carried out by reacting the compound (III) or a reactive derivative thereof with a compound having the general formula (V) in an inert solvent. The present step is carried out, for example, by the acid halide method, the mixed acid anhydride method, the active ester method or the condensation method, and is carried out in the same manner as in Step A1.

Step B2 is to prepare a compound having the general formula (I) and is carried out by reacting the compound having the general formula (VI) with a nitrating agent in the absence or presence of an inert solvent.

The nitrating agent employable here may include, for example, fuming nitric acid, nitrocollidium tetrafluoroborate, thionylchloride nitric acid, thionylnitric acid and nitronium tetrafluoroborate, and preferably fuming nitric acid, nitrocollidium tetrafluoroborate or thionylchloride nitric acid.

The inert solvent employable here is not particularly limited so long as it does not affect the reaction and may include, for example, hydrocarbons such as hexane, cyclohexane, benzene, toluene and xylene; halogenated hydrocarbons such as dichloromethane, 1,2-dichloroethane and carbon tetrachloride; ethers such as ether, tetrahydrofuran and dioxane; ketones such as acetone; nitriles such as acetonitrile; amides such as N,N-dimethylformamide, N,N-dimethylacetamide, N-methyl-2-pyrrolidone and hexamethylphosphoramide; and sulfoxides such as dimethyl sulfoxide, preferably halogenated hydrocarbons, ethers or nitriles, and particularly preferably nitriles.

The reaction temperature varies depending on the starting compound (VI), the kind of nitrating agent, etc. and is usually - 20°C to 50°C, and preferably about room temperature. The reaction time varies depending on the reaction temperature etc., and is usually 30 minutes to 24 hours (preferably 1 hour to 10 hours).

The compound (I) is also prepared by reacting the compound (VI) with a sulfonylating agent (for example, $C_1$-$C_4$ alkanesulfonyl halides such as methanesulfonyl chloride, methanesulfonyl bromide, ethanesulfonyl chloride and butanesulfonyl chloride; $C_6$-$C_{10}$ aryl halides such as benzenesulfonyl chloride, p-toluenesulfonyl chloride, p-toluenesulfonyl bromide and naphthylsulfonyl chloride; or $C_1$-$C_4$ alkanesulfonic anhydrides such as methanesulfonic anhydride, ethanesulfonic anhydride and butanesulfonic anhydride, preferably methanesulfonyl chloride, ethanesulfonyl chloride, benzenesulfonyl chloride, p-toluenesulfonyl chloride or methanesulfonic anhydride, and particularly preferably methanesulfonyl chloride) at -20°C to 50°C (preferably about room temperature) for 30 minutes to 24 hours (preferably 1 hour to 10 hours) in an inert solvent (for example, hydrocarbons such as hexane, cyclohexane, benzene, toluene and xylene; halogenated hydrocarbons such as dichloromethane, 1,2-dichloroethane and carbon tetrachloride; or ethers such as ether, tetrahydrofuran and dioxane; or nitriles such as acetonitrile, preferably nitriles, and particularly preferably acetonitrile) in the presence or absence of a base (for example, organic amines such as triethylamine, N-methylmorpholine, pyridine and 4-dimethylaminopyridine, and preferably triethylamine) to prepare the sulfonyloxy compound, and then by reacting the sulfonyloxy compound with tetra($C_1$-$C_4$ alkyl)ammonium nitrate (for example, tetramethylammonium nitrate, tetraethylammonium nitrate or tetrabutylammonium nitrate, and preferably tetrabutylammonium nitrate) at 0°C to 200°C (preferably 50°C to 150°C) for 30 minutes to 24 hours (preferably 1 hour to 10 hours) in an inert solvent (for example, aromatic hydrocarbons such as benzene, toluene and xylene, and preferably toluene).

After completion of the reaction, the desired compound of each reaction is collected from the reaction mixture according to conventional procedures. For example, the desired compound can be obtained by collecting the precipitated crystal by filtration; by distilling off the solvent; or by appropriately distilling off the solvent, adding water to the reaction mixture, extracting the mixture with a water-immiscible organic solvent such as ethyl acetate, drying the organic layer and evaporating the extracting solvent. If necessary, the compound thus obtained can be further purified by conventional procedures, for example, recrystallization, column chromatography and the like.

Method C is another method for preparing the compound (I).

Step C1 is to prepare a compound having the general formula (VIII) and is carried out by reacting a compound having the general formula (VII) or a reactive derivative thereof with the compound (V) in an inert solvent. The present step can be carried out, for example, by the acid halide method, the mixed acid anhydride method, the active ester method or the condensation method and is carried out in the same manner as in Step A1 of the above Method A.

Step C2 is to prepare a compound having the general formula (IX) and is carried out by reacting a compound having the general formula (VIII) with a nitrating agent in the absence or presence of an inert solvent. The present step is carried out in the same manner as in Step B2 of the above Method B.

Step C3 is to prepare the compound (I) and is carried out by eliminating the protective group of the mercapto group and the protective group of the amino group of the compound (IX) and then reacting the resulting compound

with carbonyl compounds such as carbonyldiimidazole; phosgene derivatives such as phosgene and triphosgene; $C_1$-$C_4$ alkyl halogenocarbonates such as methyl chlorocarbonate, ethyl chlorocarbonate, ethyl bromocarbonate, propyl chlorocarbonate and butyl chlorocarbonate; and phenyl halogenocarbonate derivatives such as phenyl chlorocarbonate, phenyl bromocarbonate, tolyl chlorocarbonate, methoxyphenyl chlorocarbonate and chlorophenyl chlorocarbonate (preferably carbonyldiimidazole, phosgene, triphosgene, methyl chlorocarbonate, ethyl chlorocarbonate, ethyl bromocarbonate or phenyl chlorocarbonate, and particularly preferably carbonyldiimidazole).

The reaction for eliminating the protective group of the mercapto group and the protective group of the amino group is carried out by the method well known in the field of synthetic organic chemistry. For example, the protective group of the mercapto group and the protective group of the amino group are eliminated by reacting a corresponding compound with an acid in an inert solvent.

The acid employable here may include, for example, mineral acis such as hydrochloric acid, nitric acid and sulfuric acid; carboxylic acids such as acetic acid and trifluoroacetic acid; and sulfonic acids such as methanesulfonic acid, benzenesulfonic acid and p-toluenesulfonic acid, preferably hydrochloric acid, trifluoroacetic acid or p-toluenesulfonic acid, and particularly preferably hydrochloric acid.

The inert solvent employable here is not particularly limited so long as it does not affect the reaction and may include, for example, hydrocarbons such as hexane, cyclohexane, benzene, toluene and xylene; halogenated hydrocarbons such as dichloromethane, 1,2-dichloroethane and carbon tetrachloride; ethers such as ether, tetrahydrofuran and dioxane; ketones such as acetone; and nitriles such as acetonitrile, preferably halogenated hydrocarbons or ethers, and particularly preferably ethers.

The reaction temperature varies depending on the starting compound etc., and is usually -20°C to 50°C, and preferably about room temperature. The reaction time varies depending on the reaction temperature etc., and is usually 30 minutes to 24 hours (preferably 1 hour to 10 hours).

In the case where the protective group of the mercapto group and/or the protective group of the amino group are a tri-substituted silyl group, the protective group is also eliminated by reacting the corresponding compound with a reagent producing a fluoro anion such as tetrabutylammonium fluoride and potassium fluoride instead of the acid.

The protective group of the mercapto group and the protective group of the amino group may be eliminated in order, and are preferably eliminated at the same time under the same condition.

The reaction of the compound obtained by eliminating the protective group of the mercapto group and the protective group of the amino group with the carbonyl compound is preferably carried out in an inert solvent.

The inert solvent employable here is not particularly limited so long as it does not affect the reaction and may include, for example, hydrocarbons such as hexane, cyclohexane, benzene, toluene and xylene; halogenated hydrocarbons such as dichloromethane, 1,2-dichloroethane and carbon tetrachloride; ethers such as ether, tetrahydrofuran and dioxane; ketones such as acetone; and nitriles such as acetonitrile, preferably halogenated hydrocarbons or ethers, and particularly preferably halogenated hydrocarbons.

The reaction temperature varies depending on the starting compound etc., and is usually -20°C to 50°C, and preferably about room temperature. The reaction time varies depending on the reaction temperature etc., and is usually 10 minutes to 10 hours (preferably 20 minutes to 5 hours).

After completion of the reaction, the desired compound of each reaction is collected from the reaction mixture by conventional procedures. For example, the desired compound can be obtained by distilling off the solvent; or by appropriately distilling off the solvent, adding water to the reaction mixture, extracting the mixture with a water-immiscible organic solvent such as ethyl acetate, drying the organic layer and evaporating the extracting solvent. If necessary, the compound thus obtained can be further purified by conventional procedures, for example, recrystallization or column chromatography.

The starting compound (VII) of Method C is known or is easily prepared according to known methods or methods which are similar thereto [for example, Chem. Absts., 74, 100379b (1971)].

A compound having the general formula (I') also has an excellent anti-ulcerative action and is prepared in the same manner as in the above process using a compound having the general formula (IV') instead of the compound (IV) or a compound having the general formula (V') instead of the compound (V).

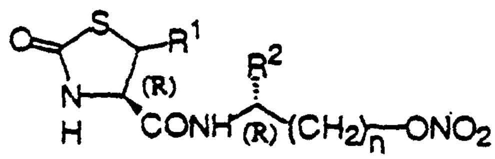

(I')

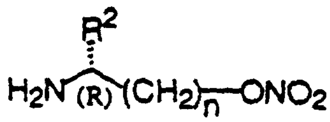

(IV')

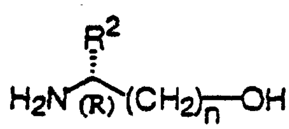

( V')

In the above formulae, $R^1$, $R^2$ and n have the same meanings as defined above.

The compound having the general formula (II) which is an active ingredient of a preventive agent or a therapeutic agent for an ulcerative disease of the present invention is a known compound or is easily prepared according to conventional procedures (for example, Japanese Unexamined Patent Publication (KOKAI) No. Hei 5-213910 etc.).

(Effect of the invention)

The compound having the above general formula (I) of the present invention exhibits a potent collateral vessel dilating action, a weak toxicity and less side effects such as headache, dizziness, tachycardia or detrimental effects on the digestive system, liver, bone etc., and it does not undergo the first-pass effect, and it is useful as a preventive agent and a therapeutic agent (preferably a therapeutic agent) for angina pectoris.

Meanwhile, the compound having the above general formula (II) or a pharmacologically acceptable salt thereof exhibits a potent anti-ulcerative action, a weak toxicity and less side effects such as headache, dizziness, tachycardia or detrimental effects on the digestive system, liver, bone etc., and it is useful as a preventive agent and a therapeutic agent (preferably a therapeutic agent) for an ulcerative disease.

The compound (I) has characteristics that its storage stability is excellent and it can be handled easily.

[Industrial applicability]

In the case where the compound (I) of the present invention is used as a therapeutic agent or a preventive agent for angina pectoris; or the compound (II) and a pharmacologically acceptable salt thereof are used as a preventive agent or a therapeutic agent for an ulcerative disease, it can be administered as such or as a mixture, for example, with a suitable pharmacologically acceptable excipient, diluent or the like in the form of a tablet, a capsule, a granule, a powder, a syrup for oral administration and an injection preparation for parenteral administration.

These preparations are prepared by the known method using additives such as excipients (for example, sugar derivatives such as lactose, sucrose, glucose, mannitol and sorbitol; starch derivatives such as corn starch, mashed potato starch, a-starch, dextrine and carboxymethyl starch; cellulose derivatives such as crystalline cellulose, low hydroxypropyl-substituted cellulose, hydroxypropylmethyl cellulose, carboxymethyl cellulose, carboxymethyl cellulose calcium and internally bridged carboxymethyl cellulose sodium; gum arabic; dextran; Pullulan; silicate derivatives such as light silicic acid anhydride, synthetic aluminum silicate and magnesium meta-silicic acid aluminate; phosphate derivatives such as calcium phosphate; carbonate derivatives such as calcium carbonate; and sulfate derivatives such as calcium sulfate), binders (for example, the above-mentioned excipients; gelatin; polyvinylpyrrolidone; and Macrogol); disintegrating agents (for example, the above-mentioned excipients; chemically modified starch, cellulose derivatives, etc. such as Crosscarmelose sodium, sodium carboxymethyl starch and bridged polyvinylpyrrolidone), lubricants (for example, talc; stearic acid; and metal stearates such as calcium stearate and magnesium stearate; colloidal silica; waxes such as beeswax and spermaceti; boric acid; glycol; carboxylic acids such as fumaric acid and adipic acid; sodium carboxylate such as sodium benzoate; sulfates such as sodium sulfate; leucine; lauryl sulfates such as sodium laurylsulfate and magnesium laurylsulfate; silicic acids such as silicic acid anhydride and silicic acid hydrate; and starch derivatives in the above excipients), stabilizers (for example, p-hydroxybenzoates such as methylparaben and propylparaben; alcohols such as chlorobutanol, benzyl alcohol and phenylethyl alcohol; benzalkonium chloride; phenols such as phenol and cresol; thimerosal; acetic anhydride; and sorbic acid); corrigents (for example, sweeteners, sour agents and perfumes conventionally used), diluents and solvents for injection agents (for example, water, ethanol and glycerin). The dose varies depending on the condition and age of the patient to be treated, and it is desirably administered 1 to 6 times daily depending on the condition: in the case of oral administration, the lower limit of 1 mg each time (preferably 5 mg) and the upper limit of 1000 mg (preferably 300 mg) for an adult; and in the case of intravenous administration, the lower limit of 0.1 mg each time (preferably 0.5 mg) and the upper-limit of 100 mg (preferably 50 mg) for an adult.

[Best mode for practicing the invention]

The present invention will be described below more specifically by showing Examples, Reference examples,

Test examples and Preparation examples, but the invention is not limited thereto.

Example 1

(4R)-N-[(1S)-1-Methyl-2-nitroxyethyl]-2-oxothiazolidin-4-vl-carboxamide (Exemplary Compound No. 1-1)

In 60 ml of dry tetrahydrofuran were suspended 2.06 g of (4R)-2-oxothiazolidine-4-carboxylic acid and 2.00 g of (1S)-1-methyl-2-nitroxyethylamine hydrochloride, 4.5 ml of triethylamine and 3.00 ml of diphenylphosphoryl azide were added thereto with stirring under ice-cooling, and the mixture was stirred at room temperature for 5 hours. Then, the solvent was distilled off under reduced pressure and the residue was purified by silica gel column chromatography employing cyclohexane-ethyl acetate (1:4) as an eluent to obtain 3.00 g of colorless crystals. The crystals were recrystallized from ethyl acetate to obtain 0.964 g of the desired compound as colorless needle crystals.

m.p.: 122-123°C (decomp.)
NMR spectrum ($CDCl_3$+DMSO-$d_6$) δ ppm: 1.27(3H, d, J=6.6Hz), 3.67 (2H, d, J=6.6Hz), 4.10-4.57(4H, m), 7.57 (1H, bs), 7.78(1H, br.s)

Example 2

(4R)-N-[(1S)-1-Methyl-2-nitroxyethyl]-2-oxothiazolidin-4-yl-carboxamide (Exemplary Compound No. 1-1)

(2a) (4R)-N-[(1S)-1-Methyl-2-hydroxyethyl]-2-oxothiazolidin-4-yl-carboxamide

In 20 ml of dry tetrahydrofuran were dissolved 1.0 g of (4R)-2-oxothiazolidine-4-carboxylic acid and 0.56 g of L-alaninol, 2.8 ml of triethylamine and 1.8 ml of diphenylphosphoryl azide were added thereto with stirring under ice-cooling, and the mixture was stirred at room temperature for 3 hours. Then, the solvent was distilled off under reduced pressure and the obtained residue was purified by silica gel column chromatography employing ethyl acetate as an eluent and further purified by silica gel column chromatography employing dichloromethanemethanol (95:5) as an eluent to obtain 0.92 g of the desired compound as colorless crystals.

m.p.: 160-162°C
NMR spectrum (DMSO-$d_6$) δ ppm: 1.04(3H, d, J=6.7Hz), 3.20-3.40(3H, m), 3.63(1H, dd, J=8.6Hz, J=11.2Hz), 3.70-3.88(1H, m), 4.16-4.22(1H, m), 4.73(1H, t, J=5.6Hz), 7.83(1H, d, J=7.9Hz), 8.24(1H, bs)

(2b) (4R)-N-[(1S)-1-Methyl-2-nitroxyethyl]-2-oxothiazolidin-4-yl-carboxamide

In 10 ml of dry acetonitrile were dissolved 478 mg of nitronium tetrafluoroboron (85% content), 0.43 ml of 2,4,6-collidine was added thereto with stirring under ice-cooling, and the mixture was stirred under ice-cooling for 30 minutes. To the resulting mixture were added 500 mg of (4R)-N-[(1S)-1-methyl-2-hydroxyethyl] -2-oxothiazolidin-4-yl-carboxamide, and the mixture was stirred at room temperature for 1 hour and 45 minutes. Then, the solvent was distilled off under reduced pressure and the thus obtained residue was purified by silica gel column chromatography employing cyclohexane-ethyl acetate (3:7) as an eluent to obtain 178 mg of the desired compound as colorless crystals.

m.p.: 119-122°C (decomp.)
NMR spectrum of the compound was identical with that of the compound of Example 1.

Example 3

(4R)-N-[(1S)-1-Methyl-2-nitroxyethyl]-2-oxothiazolidin-4-yl-carboxamide (Exemplary Compound No. 1-1)

(3a) N-[(1S)-1-Methyl-2-hydroxyethyl]-(2R)-2-t-butoxycarbonylamino-3-t-butoxycarbonylthiopropanamide

In 100 ml of dry tetrahydrofuran were dissolved 5.0 g of N,S-di-t-butoxycarbonyl-L-cysteine and 1.3 g of L-alaninol, 4.4 ml of triethylamine and 4.0 ml of diphenylphosphoryl azide were added thereto with stirring under ice-cooling, and the mixture was stirred at room temperature for 4 hours. Then, the solvent-was distilled off under reduced pressure and the residue was purified by silica gel column chromatography employing cyclohexane-ethyl acetate (1: 1) as an eluent to obtain 3.55 g of the desired compound as colorless crystals.

m.p.: 70-72°C
NMR spectrum ($CDCl_3$) δ ppm: 1.19(3H, d, J=6.9Hz), 1.45(9H, s), 1.51(9H, s), 2.65-2.90(1H, bs), 3.03-3.30(2H, m), 3.42-3.58(1H, m), 3.63-3.78(1H, m), 3.95-4.04(1H, m), 4.20-4.35(1H, m), 5.42-5.68(1H, bm), 6.46(1H, d, J=7.8Hz)

(3b) N-[(1S)-1-Methyl-2-nitroxyethyl]-(2R)-2-t-butoxycarbonylamino-3-t-butoxycarbonylthiopropanamide

In 25 ml of dry acetonitrile were dissolved 1.24 g of nitronium tetrafluoroboron (85% content), 1.12 g of 2,4,6-collidine was added thereto with stirring under ice-cooling, and the mixture was stirred under ice-cooling for 30 minutes. Then, a solution obtained by dissolving 2.5 g of N-[(1S)-1-methyl-2-hydroxyethyl] - (2R) -2-t-butoxycarbonylamino-3-t-butoxycarbonylthiopropanamide in 25 ml of dry acetonitrile was added to the mixture and the resulting mixture was stirred at room temperature for 3 hours. Then, the solvent was distilled off under reduced pressure and the thus obtained residue was purified by silica gel column chromatography employing cyclohexane-ethyl acetate (4:1) as an eluent to obtain 1.39 g of the desired compound as pale yellow crystals.

m.p.: 123-124°C (decomp.)
NMR spectrum ($CDCl_3$) δ ppm: 1.27(3H, d, J=6.8Hz), 1.45(9H, s), 1.51(9H, s), 3.05-3.28(2H, m), 4.20-4.55(4H, m), 5.42(1H, d, J=6.3Hz), 6.45-6.65(1H, bs)

(3c) (4R)-N-1(1*S*)-1-Methyl-2-nitroxyethyl]-2-oxothiazolidin-4-yl-carboxamide

In 10 ml of a solution of 4N hydrochloric acid in dioxane were dissolved 1.0 g of N-[(1*S*)-1-methyl-2-nitroxyethyl]-(2R)-2-t-butoxycarbonylamino-3-t-butoxycarbonylthiopropanamide, and the solution was stirred at room temperature for 2 hours. The solvent was distilled off under reduced pressure, benzene was added to the residue, and the solution was evaporated to dryness under reduced pressure. The thus obtained residue was suspended in 10 ml of dry dichloromethane, 0.46 g of carbodiimidazole were added thereto, and the mixture was stirred at room temperature for 40 minutes. The reaction mixture was purified by silica gel column chromatography employing ethyl acetate as an eluent to obtain 0.45 g of pale yellow crystals. The crystals were recrystallized from ethyl acetate to obtain 84 mg of the desired compound as colorless crystals.

m.p.: 125-126°C (decomp.)
NMR spectrum of the compound was identical with that of the compound of Example 1.

Example 4

(4R)-N-[(1S)-1-Ethyl-2-nitroxyethyl]-2-oxothiazolidin-4-yl-carboxamide (Exemplary Compound No. 1-15)

In 10 ml of dry tetrahydrofuran were suspended 0.50 g of (4R)-2-oxothiazolidine-4-carboxylic acid and 0.70 g of (1S)-1-ethyl-2-nitroxyethylamine hydrochloride, 1.40 ml of triethylamine and 0.62 ml of diethylcyanophosphoric acid were added thereto, and the mixture was stirred at room temperature for 2 hours. The solvent was distilled off under reduced pressure and the residue was purified by silica gel column chromatography employing cyclohexane-ethyl acetate (1:2) as an eluent to obtain a pale yellow oil. Isopropyl ether was added to the oil to obtain a pale yellow powder. The powder was dissolved in 10 ml of acetone and further 5 ml of ethyl acetate was added thereto. The acetone was distilled off under reduced pressure and the mixture was left to stand at room temperature to obtain 0.24 g of the desired compound as colorless columnar crystals.

m.p.: 106-107°C (decomp.)
NMR spectrum ($d_6$-DMSO) δ ppm: 0.87(3H, t, J=7.4Hz), 1.30-1.68(2H, m), 3.32(1H, dd, J=5.0Hz, J=11.2Hz), 3.68 (1H, dd, J=8.5Hz, J=11.2Hz), 3.95-4.10(1H, m), 4.25-4.35(1H, m), 4.41(1H, dd, J=7.5Hz, J=11.2Hz), 4.60(1H, dd, J=4.3Hz, J=11.2Hz), 8.09(1H, d, J=8.5Hz), 8.27(1H, bs)

Example 5

(4R)-N-[(1S)-1-Propyl-2-nitroxyethyl]-2-oxothiazolidin-4-yl-carboxamide (Exemplary Compound No. 1-27)

0.84 g of the desired compound was obtained as colorless crystals in similar procedures to those in Example 4 by using 0.50 g of (4R)-2-oxothiazolidine-4-carboxylic acid and 0.75 g of (15)-1-propyl-2-nitroxyethylamine hydrochloride.

m.p.: 99-100°C (decomp.)
NMR spectrum ($d_6$-DMSO) δ ppm: 0.87(3H, t, J=7.1Hz), 1.15-1.55(4H, m), 3.32(1H, dd, J=4.8Hz, J=11.2Hz), 3.68 (1H, dd, J=8.6Hz, J=11.2Hz), 4.03-4.18(1H, m), 4.23-4.33(1H, m), 4.39(1H, dd, J=7.5Hz, J=11.2Hz), 4.60(1H, dd, J=4.2Hz, J=11.2Hz), 8.09(1H, d, J=8.5Hz), 8.27(1H, bs)

Example 6

(4R)-N-[(1S)-1-Butyl-2-nitroxyethyl]-2-oxothiazolidin-4-yl-carboxamide (Exemplary Compound No. 1-71)

570 mg of the desired compound were obtained as colorless crystals in similar procedures to those in Example 4 by using 441 mg of (4R)-2-oxothiazolidine-4-carboxylic acid and 500 mg of (1S)-1-butyl-2-nitroxyethylamine hydrochloride.

m.p.: 110-112°C (decomp.)
NMR spectrum ($CDCl_3$) δ ppm: 0.91(3H, t, J=7.2Hz), 1.20-1.43(4H, m), 1.45-1.75(2H, m), 3.61(1H, dd, J=4.9Hz, J=11.2Hz), 3.83(1H, dd, J=8.6Hz, J=11.2Hz), 4.23-4.45(3H, m), 4.60(1H, dd, J=3.3Hz, J=11.2Hz), 6.65(1H, bs), 6.81(1H, d, J=8.6Hz)

Example 7

(4R)-N-[(1S)-1-Isopropyl-2-nitroxvethyl]-2-oxothiazolidin-4-yl-carboxamide (Exemplary Compound No. 1-59)

343 mg of the desired compound were obtained as colorless crystals in similar procedures to those in Example 4 by using 333 mg of (4R)-2-oxothiazolidine-4-carboxylic acid and 500 mg of (1S)-1-isopropyl-2-nitroxyethylamine hydrochloride.

m.p.: 89-91°C
NMR spectrum ($d_6$-DMSO) δ ppm: 0.96(3H, d, J=7.3Hz), 1.00(3H, d, J=6.6Hz), 1.83-2.02(1H, m), 3.64(1H, dd, J=4.0Hz, J=11.2Hz), 3.84(1H, dd, J=8.6Hz, J=11.2Hz), 4.05-4.20(1H, m), 4:35-4.52(2H, m), 4.65(1H, dd, J=4.0Hz, J=11.2Hz), 6.80(1H, bs), 6.86(1H, d, J=9.2Hz)

Example 8

(4R)-N-[(1S)-1-Isobutyl-2-nitroxyethyl]-2-oxothiazolidin-4-yl-carboxamide (Exemplary Compound No. 1-83)

636 mg of the desired compound were obtained as yellow oil in similar procedures to those in Example 4 by using 544 mg of (4R)-2-oxothiazolidine-4-carboxylic acid and 500 mg of (1S)-1-isobutyl-2-nitroxyethylamine hydrochloride.

NMR spectrum ($d_6$-DMSO) δ ppm: 0.93(3H, d, J=6.6Hz), 0.95(3H, d, J=7.9Hz), 1.30-1.80(3H, m), 3.61(1H, dd, J=4.0Hz, J=11.2Hz), 3.82(1H, dd, J=8.6Hz, J=11.2Hz), 4.30-4.50(3H, m), 4.59(1H, dd, J=3.3Hz, J=11.2Hz), 6.69(1H, bs), 6.85(1H, d, J=7.9Hz)

Reference example 1

(4R)-N-[(1R)-1-Methyl-2-nitroxyethyl]-2-oxothiazolidin-4-yl-carboxamide

In 100 ml of dry benzene were suspended 5.64 g of (4R)-2-oxothiazolidine-4-carboxylic acid, 6.7 ml of oxalyl chloride and a few drops of dimethylformamide were added thereto, and the mixture was stirred at room temperature for 3 hours. The solvent was distilled off under reduced pressure, further benzene was added thereto, and the suspension was distillated azeotropically to dryness to obtain the pale yellow acid chloride.
In 150 ml of dry dichloromethane were suspended 5.00 g of (1R)-1-methyl-2-nitroxyethylamine hydrochloride, 14 ml of triethylamine and a solution of the previously obtained acid chloride in 70 ml of dry dichloromethane were added dropwise thereto with stirring under ice-cooling, and the mixture was stirred under ice-cooling for 1 hour.
Then, the solvent was distilled off under reduced pressure and the residue was purified by silica gel column chromatography employing cyclohexane-ethyl acetate (1:4) as an eluent to obtain pale yellow crystals. The crystals were recrystallized from ethyl acetate to obtain 2.79 g of the desired compound as colorless crystals.

m.p.: 101-102°C (decomp.)

NMR spectrum ($CDCl_3$+$d_6$-DMSO) δ ppm: 1.27(3H, d, J=6.9Hz), 3.55-3.75(2H, m), 4.23-4.58(4H, m), 7.47(1H, d, J=7.1Hz), 7.61(1H, s)

Reference example 2

(1S)-N-(t-Butoxycarbonyl)-1-methyl-2-nitroxyethylamine

In 200 ml of dry acetonitrile were suspended 17.9 g of nitronium tetrafluoroborate, and 17.5 ml of 2,4,6-collidine were added dropwise thereto at -5°C to 0°C under a nitrogen stream. The reaction mixture was stirred at 0°C for 30 minutes, 10.7 g of N-t-butoxycarbonyl-L-alaninol were added thereto, and the mixture was stirred at room temperature for 1 hour and 20 minutes. Then, the solvent was distilled off under reduced pressure and ethyl acetate was added to the residue. The insolubles were filtered off and the filtrate was evaporated to dryness under reduced pressure. The thus obtained yellow oil was purified by silica gel column chromatography employing cyclohexane-ethyl acetate (9:1) as an eluent to obtain 7.12 g of the desired compound as a colorless oil.
NMR spectrum ($CDCl_3$) δ ppm: 1.23(3H, d, J=7.3Hz), 1.45(9H, s), 3.90-4.15 (1H, m), 4.27-4.75 (3H, m)

Reference example 3

(1S)-1-Methyl-2-nitroxyethylamine hydrochloride

In 80 ml of 4N hydrochloric acid-dioxane were dissolved 4.52 g of (1S)-N- (t-butoxycarbonyl)-1-methyl-2-nitroxyethylamine, and the mixture was left to stand at room temperature for 1 hour and 50 minutes. To the mixture were added 160 ml of ether, and the crystals were collected by filtration and dried to obtain 3.02 g of the desired compound as colorless crystals.

m.p.: 134-135°C
NMR spectrum ($CDCl_3$+$d_6$-DMSO) δ ppm: 1.47(3H, d, J=6.6Hz), 3.55-3.70(1H, m), 4.65-4.80(2H, m)

Reference example 4

(1R)-N-(t-Butoxycarbonyl)-1-methyl-2-nitroxyethylamine

8.55 g of the desired compound were obtained as colorless oil in similar procedures to those in Reference example 2 by using 13.09 g of nitronium tetrafluoroborate and 7.72 g of N-t-butoxycarbonyl-D-alaninol.
NMR spectrum ($CDCl_3$) δ ppm: 1.23(3H, d, J=7.3Hz), 1.45(9H, s), 3.95-4.15(1H, m), 4.28-4.75(3H, m)

Reference example 5

(1R)-1-Methyl-2-nitroxyethylamine hydrochloride

1.60 g of the desired compound were obtained as colorless crystals in similar procedures to those in Reference example 3 by using 8.55 g of (1R)-N-(t-butoxycarbonyl)-1-methyl-2-nitroxyethylamine and 90 ml of 4N hydrochloric acid-dioxane.

m.p.: 133-135°C
NMR spectrum ($CDCl_3$+DMSO-$d_6$) δ ppm: 1.47(3H, d, J=6.9Hz), 3.55-3.70 (1H, m), 4.65-4.78(2H, m)

Reference example 6

(1S)-N-(t-Butoxycarbonyl)-1-ethyl-2-nitroxyethylamine

3.19 g of the desired compound were obtained as a pale yellow oil in similar procedures to those in Reference example 2 by using 4.00 g of nitronium tetrafluoroborate and 4.03 g of (1S)-N-(t-butoxycarbonyl)-1-ethyl-2-hydroxyethylamine.
NMR spectrum ($CDCl_3$) δ ppm: 0.98(3H, t, J=7.3Hz), 1.40-1.70(2H, m), 1.45(9H, s), 3.70-3.95(1H, m), 4.20-4.70 (3H, m)

Reference example 7

(1*S*)-1-Ethyl-2-nitroxyethylamine hydrochloride

2.10 g of the desired compound were obtained as colorless crystals in similar procedures to those in Reference example 3 by using 3.19 g of (1S)-N-(t-butoxycarbonyl)-1-ethyl-2-nitroxyethylamine and 50 ml of 4N hydrochloric acid-dioxane.

m.p.: 121-123°C (decomp.)
NMR spectrum ($d_6$-DMSO) δ ppm: 0.96(3H, t, J=7.2Hz), 1.50-1.80(2H, m), 3.35-3.50(1H, m), 4.66(1H, dd, J=6.6Hz, J=11.9Hz), 4.81(1H, dd, J=4.0Hz, J=11.9Hz), 8.49(3H, bs)

Reference example 8

(1S)-N-(t-Butoxycarbonyl)-1-propyl-2-nitroxyethylamine

3.03 g of the desired compound were obtained as colorless crystals in similar procedures to those in Reference example 2 by using 8.60 g of nitronium tetrafluoroborate and 7.49 g of (1S)-N-(t-butoxycarbonyl)-1-propyl-2-hydroxyethylamine.

m.p.: 57-58°C
NMR spectrum ($CDCl_3$) δ ppm: 0.95(3H, t, J=7.0Hz), 1.25-1.70(4H, m), 1.45(9H, s), 3.80-4.05(1H, m), 4.20-4.70 (3H, m)

Reference example 9

(1S)-1-Propyl-2 -nitroxyethylamine hydrochloride

2.77 g of the desired compound were obtained as colorless crystals in similar procedures to those in Reference example 3 by using 4.00 g of (1S)-N-(t-butoxycarbonyl)-1-propyl-2-nitroxyethylamine and 40 ml of 4N hydrochloric acid-dioxane.

m.p.: 157-158°C (decomp.)
NMR spectrum ($d_6$-DMSO) δ ppm: 0.89(3H, t, J=7.2Hz), 1.30-1.70(4H, m), 3.40-3.55(1H, m), 4.65(1H, dd, J=6.8Hz, J=11.9Hz), 4.81(1H, dd, J=3.4Hz, J=11.9Hz), 8.51(3H, bs)

Reference example 10

(1S)-N-(t-Butoxycarbonyl)-1-butyl-2-nitroxyethylamine

1.56 g of the desired compound were obtained as a yellow oil in similar procedures to those in Reference example 2 by using 1.87 g of nitronium tetrafluoroborate and 2.09 g of (1S)-N-(t-butoxycarbonyl)-1-butyl-2-hydroxyethylamine.

NMR spectrum ($CDCl_3$) δ ppm: 0.91(3H, t, J=7.3Hz), 1.25-1.65(6H, m), 1.45(9H, s), 3.83-3.98(1H, m), 4.30-4.60(3H, m)

Reference example 11

(1S)-1-Butyl-2-nitroxyethylamine hydrochloride

702 mg of the desired compound were obtained as colorless crystals in similar procedures to those in Reference example 3 by using 1.56 g of (1S)-N-(t-butoxycarbonyl)-1-butyl-2-nitroxyethylamine and 15 ml of 4N hydrochloric acid-dioxane.

m.p.: 133-135°C (decomp.)
NMR spectrum ($CDCl_3$) δ ppm: 0.94(3H, t, J=7.3Hz), 1.20-2.00(6H, m), 3.55-3.70(1H, m), 4.65-4.85(2H, m)

Reference example 12

(1S)-N-(t-Butoxycarbonyl)-1-isopropyl-2-nitroxyethylamine

3.07 g of the desired compound were obtained as a yellow oil in similar procedures to those in Reference example 2 by using 3.19 g of nitronium tetrafluoroborate and 3.31 g of (1S)-N-(t-butoxycarbonyl)-1-isopropyl-2-hydroxyethylamine.

NMR spectrum ($CDCl_3$) δ ppm: 0.97(3H, d, J=5.9Hz), 0.99(3H, d, J=6.6Hz), 1.45(9H, s), 3.65-3.80(1H, m), 4.35-4.63(3H, m)

Reference example 13

(15)-1-Isopropyl-2-nitroxyethylamine hydrochloride

1.97 g of the desired compound were obtained as colorless crystals in similar procedures to those in Reference example 3 by using 3.07 g of (1S)-N-(t-butoxycarbonyl)-1-isopropyl-2-nitroxyethylamine and 30 ml of 4N hydrochloric acid-dioxane.

m.p.: 174-175°C (decomp.)
NMR spectrum ($CDCl_3$) δ ppm: 1.14(3H, d, J=7.3Hz), 1.17(3H, d, J=6.6Hz), 2.10-2.30(1H, m), 3.40-3.52(1H, m), 4.70-4.90(2H, m)

Reference example 14

(1S)-N-(t-Butoxycarbonyl)-1-isobutyl-2-nitroxyethylamine

3.84 g of the desired compound were obtained as a yellow oil in similar procedures to those in Reference example 2 by using 3.91 g of nitronium tetrafluoroborate and 4.35 g of (1S)-N-(t-butoxycarbonyl)-1-isobutyl-2-hydroxyethylamine.

NMR spectrum ($CDCl_3$) δ ppm: 0.93(3H, d, J=4.6Hz), 0.95(3H, d, J=4.6Hz), 1.20-1.50(2H, m), 1.45(9H, s), 1.60-1.80(1H, m), 3.90-4.10(1H, m), 4.25-4.65(3H, m)

Reference example 15

(1S) -1-Isobutyl-2-nitroxyethylamine hydrochloride

2.32 g of the desired compound were obtained as colorless crystals in similar procedures to those in Reference example 3 by using 3.84 g of (1S)-N-(t-butoxycarbonyl)-1-isobutyl-2-nitroxyethylamine and 40 ml of 4N hydrochloric acid-dioxane.

m.p.: 174-175°C (decomp.)
NMR spectrum ($CDCl_3$) δ ppm: 0.93-1.10(6H, m), 1.50-1.70(1H, m), 1.72-2.00(2H, m), 3.65-3.82(1H, m), 4.63-4.85 (2H, m)

(Test example 1)

Collateral Vessel Dilating Action by Intravenous Administration

Beagle dogs (male) weighing 9 to 13 kg were anesthetized by intravenous injection of pentobarbital at a dose of 30 mg/kg and tested under artificial respiration. In order to measure the pressure of the left carotid artery, a polyethylene cannula (Atom intravenous catheter 2F) was inserted antegrade into one branch of the left thyroid artery. The left carotid artery upstream from this pressure measurement site was occluded for 1 minute with an arterial clamp, and the pressure immediately before occlusion (P) and the decrease in peripheral pressure (ΔP) were measured. Next, the test drug was administered through another polyethylene cannula inserted into the inguinal vein. The left carotid artery was occluded for 1 minute after 5, 15, 30, 45 and 60 minutes, and pressure immediately before occlusion (Pa) and the decrease in peripheral pressure (ΔPa) each time were measured. Collateral vessel dilating effect (Collateral Index = CI) of the test drug was determined according to the following formula:

$$CI = 100 - (\Delta Pa/Pa) \times 100/(\Delta P/P)$$

As a result of this test, the compounds of Examples 1, 5, 6 and 8 exhibited an excellent action, CI(60) at the dose of 0.3 mg/kg being more then 10.

(Test example 2)

Collateral Vessel Dilating Action by Administration into the Portal Vein

While test specimens were prepared according to the method of Test example 1, the animal was laparotomized along the abdominal median line, and a branch of the mesenteric vein was removed and incised so as to administer the test drug into the portal vein. A polyethylene cannula (Atom intravenous catheter 2F) was inserted antegrade into this vein to reside in the portal vein, and then the test drug was administered through it. In order to test the first-pass effect of the test drug, it was first administered intravenously (inguinal vein) to determine collateral vessel dilating action of the drug for 60 minutes. The same test drug was then administered into the portal vein 2 or 3 hours later to determine collateral vessel dilating action for 60 minutes, and those actions were compared with each other.

As a result of this test, the compound of Example 1 exhibited an excellent collateral vessel dilating action.

(Test example 3)

Inhibition of aspirin-induced ulcer

As a test animal, 10 Donryu strain male rats each weighing 200 g-250 g were used in one group. The rats were fasted before the experiment for 24 hours but they could freely drink water. The test compounds were suspended in 0.5% carboxymethyl cellulose (CMC) solution. Meanwhile, for a control group, 0.5% CMC solution was used.

The test compound was orally administered (0.1 ml/100 g body weight) to the rats and an aspirin solution (150 mg/ml: suspended in 0.5% CMC solution) was orally administered (0.1 ml/100 g body weight) to the rats 1 hour later. Four hours after the administration of the aspirin solution, the rats were sacrificed using carbon dioxide gas and the stomach of each rat was taken out. Into the stomachs were poured 10 ml of 1% formalin solution to allow the stomachs to expand, and the stomachs were immersed in 1% formalin solution in a beaker for about 15-20 minutes. Then, each stomach was cut along the greater curvature thereof and an area of the ulceration in the gastric mucosa was measured by means of an image analysis apparatus [Luzex-F: manufactured by Nireko Co., Ltd.]. The average value of the area of the ulceration of each group was calculated from the area of the ulceration of each rat and the inhibition rate was obtained by comparing the average value of the test group with that of the control group. The results are shown in Table 4.

[Table 4]

| Compound | Administered dose (mg/kg) | Inhibition rate (%) |
|---|---|---|
| Compound of Example 1 | 30 | 63.58*) |
| | 100 | 80.15**) |
| | 300 | 93.17**) |

*) $p<0.05$
**) $p<0.005$

According to the present test, the compound of Example 1 exhibited excellent anti-ulcerative action.

(Test Example 4)

Stability of Compounds

About 2 mg of the test compound was accurately weighed and placed in a brown bottle. The bottle was left to stand at a dark place at room temperature (24-26°C) for 4 weeks and a residual ratio (%) of the test compound was measured by high pressure liquid chromatography (column: Inertsil ODS-3, eluting solvent: 10 mM (pH=7.0) phosphoric acid buffer/acetonitrile=80/20). The results are shown in Table 5.

[Table 5]

| Compound | Residual ratio (%) |
| --- | --- |
| Compound of Example 1 | 101.2 |
| Compound A*) | 49.8**) |

*) 1:1 Mixture of compound of Example 1 and the (4R),(1R)-isomer thereof

**) Compound of Example 1/the (4R),(1R)-isomer thereof = 2/1

According to the present test, the compound of Example 1 exhibited excellent storage stability as compared with that of 1:1 mixture of the compound of Example 1 and the (4R),(1R)-isomer thereof. Meanwhile, the compound of Example 1 exhibited excellent storage stability as compared with the (4R), (1R)-isomer.

(Preparation example 1)

| Capsule | |
| --- | --- |
| Compound of Example 1 | 50.0 mg |
| Lactose | 128.7 |
| Corn starch | 70.0 |
| Magnesium stearate | 1.3 |
| | 250 mg |

The thus formulated powder is mixed and passes through a sieve of 60 mesh, and then the powder is encapsulated in No. 3 gelatin capsule of 250 mg to prepare a capsule.

(Preparation example 2)

| Tablet | |
| --- | --- |
| Compound of Example 1 | 50.0 mg |
| Lactose | 124.0 |
| Corn starch | 25.0 |
| Magnesium stearate | 1.0 |
| | 200 mg |

The thus formulated powder is mixed and a 200 mg-tablet is made by means of a tablet making machine.
If necessary, sugar coating can be applied to the tablet.

## Claims

1. An optically active thiazolidinone derivative having the general formula:

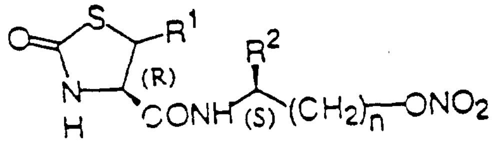

(I)

wherein:

$R^1$ represents a hydrogen atom, an alkyl group having from 1 to 4 carbon atoms, a phenyl group which is unsubstituted or is substituted by a substituent selected from the group consisting of alkyl groups having from 1 to 4 carbon atoms, alkoxy groups having from 1 to 4 carbon atoms and halogen atoms, or a phenylalkyl group wherein the alkyl group has 1 or 2 carbon atoms and the phenyl group is unsubstituted or is substituted by a substituent selected from the group consisting of alkyl groups having from I to 4 carbon atoms, alkoxy groups having from 1 to 4 carbon atoms and halogen atoms;

$R^2$ represents an alkyl group having from 1 to 6 carbon atoms; and

n represents 1 or 2.

**2.** The optically active thiazolidinone derivative according to claim 1, wherein $R^1$ is a hydrogen atom, an alkyl group having from 1 to 4 carbon atoms, a phenyl group which is unsubstituted or is substituted by a substituent selected from methyl groups, methoxy groups, fluorine atoms and chlorine atoms, a benzyl group which is unsubstituted or is substituted by a substituent selected from methyl groups, methoxy groups, fluorine atoms and chlorine atoms, or a phenethyl group which is unsubstituted or is substituted by a substituent selected from methyl groups, methoxy groups, fluorine atoms and chlorine atoms.

**3.** The optically active thiazolidinone derivative according to claim 1, wherein $R^1$ is a hydrogen atom, a methyl group, a 4-methoxyphenyl group or a benzyl group.

**4.** The optically active thiazolidinone derivative according to claim 1, wherein $R^1$ is a hydrogen atom.

**5.** The optically active thiazolidinone derivative according to claim 1, wherein $R^2$ is an alkyl group having from 1 to 4 carbon atoms.

**6.** The optically active thiazolidinone derivative according to claim 1, wherein $R^2$ is a methyl group, an ethyl group, a propyl group, an isopropyl group, a butyl group or an isobutyl group.

**7.** The optically active thiazolidinone derivative according to claim 1, wherein $R^2$ is a methyl group, a propyl group, a butyl group or an isobutyl group.

**8.** The optically active thiazolidinone derivative according to claim 1, wherein $R^2$ is a methyl group.

**9.** The optically active thiazolidinone derivative according to claim 1, wherein n is 1.

**10.** The optically active thiazolidinone derivative according to claim 1, wherein:

$R^1$ is a hydrogen atom, an alkyl group having from 1 to 4 carbon atoms, a phenyl group which is unsubstituted or is substituted by a substituent selected from methyl groups, methoxy groups, fluorine atoms and chlorine atoms, a benzyl group which is unsubstituted or is substituted by a substituent selected from methyl groups, methoxy groups, fluorine atoms and chlorine atoms, or a phenethyl group which is unsubstituted or is substituted by a substituent selected from methyl groups, methoxy groups, fluorine atoms and chlorine atoms;
$R^2$ is an alkyl group having from 1 to 4 carbon atoms; and
n is 1.

**11.** The optically active thiazolidinone derivative according to claim 1, wherein:

$R^1$ is a hydrogen atom, a methyl group, a 4-methoxyphenyl group or a benzyl group;
$R^2$ is a methyl group, an ethyl group, a propyl group, an isopropyl group, a butyl group or an isobutyl group; and
n is 1.

**12.** The optically active thiazolidinone derivative according to claim 1, wherein:

$R^1$ is a hydrogen atom;
$R^2$ is a methyl group, a propyl group, a butyl group or an isobutyl group; and
n is 1.

**13.** The optically active thiazolidinone derivative according to claim 1, wherein $R^1$ is a hydrogen atom and $R^2$ is a methyl group.

**14.** The optically active thiazolidinone derivative according to claim 1, selected from the group consisting of:

(4R)-N-[(1S)-1-methyl-2-nitroxyethyl]-2-oxothiazolidin-4-yl-carboxamide.
(4R)-N-[(1S)-1-methyl-2-nitroxyethyl]-5-methyl-2-oxothiazolidin-4-yl-carboxamide;
(4R)-N-[(1S)-1-methyl-2-nitroxyethyl]-5-(4-methoxyphenyl)-2-oxothiazolidin-4-yl-carboxamide;
(4R)-N-[(1S)-1-methyl-2-nitroxyethyl]-5-benzyl-2-oxothiazolidin-4-yl-carboxamide;
(4R)-N-[(1S)-1-ethyl-2-nitroxyethyl]-2-oxothiazolidin-4-yl-carboxamide;
(4R)-N-[(1S)-1-Propyl-2-nitroxyethyl]-2-oxothiazolidin-4-yl-carboxamide;
(4R)-N-[(1S)-1-propyl-2-nitroxyethyl]-5-methyl-2-oxothiazolidin-4-yl-carboxamide;
(4R)-N-[(1S)-1-propyl-2-nitroxyethyl]-5-(4-methoxyphenyl)-2-oxothiazolidin-4-yl-carboxamide;
(4R)-N-[(1S)-1-propyl-2-nitroxyethyl]-5-benzyl-2-oxothiazolidin-4-yl-carboxamide;
(4R)-N-[(1S)-1-butyl-2-nitroxyethyl]-2-oxothiazolidin-4-yl-carboxamide;
(4R)-N-[(1S)-1-butyl-2-nitroxyethyl]-5-methyl-2-oxothiazolidin-4-yl-carboxamide;
(4R)-N-[(1S)-1-butyl-2-nitroxyethyl]-5-(4-methoxyphenyl)-2-oxothiazolidin-4-yl-carboxamide;
(4R)-N-[(1S)-1-butyl-2-nitroxyethyl]-5-benzyl-2-oxothiazolidin-4-yl-carboxamide;
(4R)-N-[(1S)-1-isobutyl-2-nitroxyethyl]-2-oxothiazolidin-4-yl-carboxamide;
(4R)-N-[(1S)-1-isobutyl-2-nitroxyethyl]-5-methyl-2-oxothiazolidin-4-yl-carboxamide;
(4R)-N-[(1S)-1-isobutyl-2-nitroxyethyl]-5-(4-methoxyphenyl)-2-oxothiazolidin-4-yl-carboxamide; and
(4R)-N-[(1S)-1-isobutyl-2-nitroxyethyl]-5-benzyl-2-oxothiazolidin-4-yl-carboxamide;

**15.** A composition for preventing or treating angina pectoris comprising an effective amount of an active compound in admixture with a pharmacologically acceptable carrier or diluent, wherein said active compound is an optically active thiazolidinone derivative according to any one of claims 1 to 14.

**16.** An optically active thiazolidinone derivative according to any one of claims 1 to 14 for use as a medicament.

**17.** The use of an optically active thiazolidinone derivative according to any one of claims 1 to 14 in the manufacture of a medicament for the prevention or treatment of angina pectoris.

**18.** A process for preparing an optically active thiazolidinone derivative according to any one of claims 1 to 14 comprising reacting a carboxylic acid having the general formula:

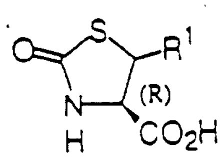

(III)

wherein $R^1$ is as defined in claim 1,
or a reactive derivative thereof with an amine having the general formula:

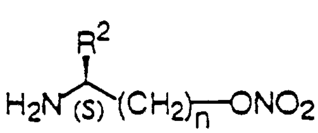

(IV)

wherein $R^2$ is as defined in claim 1, or an acid addition salt thereof.

**19.** A process for preparing an optically active thiazolidinone derivative according to any one of claims 1 to 14 comprising reacting a carboxylic acid having the general formula:

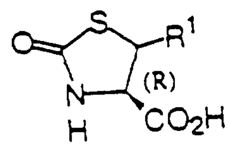

(III)

wherein $R^1$ is as defined in claim 1,
or a reactive derivative thereof with an amine compound having the general formula:

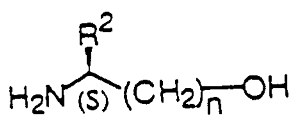

(V)

wherein $R^2$ is as defined in claim 1,
or an acid addition salt thereof and then nitrating the compound thus obtained.

**20.** The use of a thia- or oxazolidinone derivative having the general formula (II) below, or a pharmacologically acceptable salt thereof, in the manufacture of a medicament for the treatment or prevention of ulcerative disease:

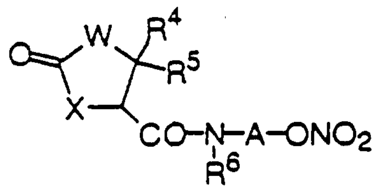

(II)

wherein:

W represents a sulfur atom or an oxygen atom and X represents a group having the formula-$N(R^3)$-, or X represents a sulfur atom or an oxygen atom and W represents a group having the formula -$N(R^3)$-;
$R^3$ represents a hydrogen atom, an alkyl group having from 1 to 6 carbon atoms or an aralkyl group wherein the alkyl group has from I to 4 carbon atoms and the aryl group is as defined below;
$R^4$ and $R^5$ are the same or different and each represents a hydrogen atom, an alkyl group having from 1 to 6 carbon atoms, an aralkyl group wherein the alkyl group has from 1 to 4 carbon atoms and the aryl group is as defined below, an aryl group as defined below, a 5- or 6-membered aromatic heterocyclic group containing I to 3 heteroatoms selected from the group consisting of nitrogen atoms, oxygen atoms and sulfur atoms which may be optionally condensed with a benzene ring or a 5- or 6-membered aromatic heterocyclic group containing 1 to 3 heteroatoms selected from the group consisting of nitrogen atoms, oxygen atoms and sulfur atoms which may be optionally substituted and optionally condensed with a benzene ring, said optional substituents being selected from the group consisting of alkyl groups having from 1 to 6 carbon atoms, amino groups and mono- and di-alkylamino groups wherein the or each alkyl group has from 1 to 6 carbon atoms;
$R^6$ represents a hydrogen atom, an alkyl group having from 1 to 6 carbon atoms or an aralkyl group wherein the alkyl group has from 1 to 4 carbon atoms and the aryl group is as defined below;
A represents an alkylene group which has from 2 to 6 carbon atoms and which is unsubstituted or is substituted by a substituent selected from the group consisting of carboxyl groups, alkoxycarbonyl groups wherein the alkoxy groups have from 1 to 6 carbon atoms and aryloxycarbonyl groups wherein the aryl group is as defined below; the above-mentioned aryl groups have from 6 to 10 carbon atoms and may be unsubstituted or may be substituted by a substituent selected from the group consisting of alkyl groups having from 1 to 6 carbon

atoms, alkoxy groups having from 1 to 6 carbon atoms, hydroxy groups, halogen atoms, amino groups, mono- and di-alkylamino groups wherein the or each alkyl group has from 1 to 6 carbon atoms and nitro groups.

**21.** The use according to claim 20, wherein $R^3$ is a hydrogen atom, an alkyl group having from 1 to 4 carbon atoms, a benzyl group or a phenethyl group.

**22.** The use according to claim 20, wherein $R^3$ is a hydrogen atom, a methyl group or a benzyl group.

**23.** The use according to claim 20, wherein:

W is a sulfur atom or an oxygen atom and X is a group having the formula $-NR^3-$, wherein $R^3$ is a hydrogen atom; or
X is a sulfur atom and W is a group having the formula $-NR^3-$, wherein $R^3$ is a hydrogen atom.

**24.** The use according to claim 20, wherein W is a sulfur atom or an oxygen atom and X is a group having the formula $-NR^3-$, wherein $R^3$ is a hydrogen atom.

**25.** The use according to claim 20, wherein $R^4$ and $R^5$ are the same or different and each is a hydrogen atom, an alkyl group group having from 1 to 4 carbon atoms, a phenylalkyl group wherein the alkyl group has from 1 to 4 carbon atoms and the phenyl group is unsubstituted or is substituted by a substituent selected from the group consisting of alkyl groups having from 1 to 4 carbon atoms, alkoxy groups having from 1 to 4 carbon atoms, hydroxy groups, halogen atoms and nitro groups, a naphthylmethyl group, a phenyl group which is unsubstituted or is substituted by a substituent selected from the group consisting of alkyl groups having from 1 to 4 carbon atoms, alkoxy groups having from 1 to 4 carbon atoms, hydroxy groups, halogen atoms and nitro groups, a naphthyl group or a heterocyclic group selected from furyl, thienyl, pyridyl, oxazolyl, thiazolyl, isoxazolyl and isothiazolyl groups, said group being unsubstituted or substituted by an alkyl group having from 1 to 4 carbon atoms.

**26.** The use according to claim 20, wherein $R^4$ and $R^5$ are the same or different and each is a hydrogen atom, a methyl group, a benzyl group which is unsubstituted or is substituted by a substituent selected from the group consisting of methyl groups, methoxy groups, hydroxy groups, fluorine atoms and chlorine atoms, a phenethyl group which is unsubstituted or is substituted by a substituent selected from the group consisting of methyl groups, methoxy groups, hydroxy groups, fluorine atoms and chlorine atoms, a phenyl group which is unsubstituted or is substituted by a substituent selected from the group consisting of methyl groups, methoxy groups, hydroxy groups, fluorine atoms and chlorine atoms, a furyl group, a thienyl group or a pyridyl group.

**27.** The use according to claim 20, wherein:

$R^4$ is a hydrogen atom, a methyl group, a benzyl group which is unsubstituted or is substituted by a substituent selected from the group consisting of methyl groups, methoxy groups and hydroxy groups, or a phenyl group which is unsubstituted or is substituted by a substituent selected from the group consisting of methyl groups, methoxy groups and hydroxy groups; and
$R^5$ is a hydrogen atom.

**28.** The use according to claim 20, wherein:

$R^4$ is a hydrogen atom, a methyl group, a benzyl group, a phenyl group or a methoxyphenyl group; and
$R^5$ is a hydrogen atom.

**29.** The use according to claim 20, wherein:

$R^4$ is a hydrogen atom, a methyl group, a benzyl group or a 4-methoxyphenyl group; and
$R^5$ is a hydrogen atom.

**30.** The use according to claim 20, wherein $R^4$ is a hydrogen atom and $R^5$ is a hydrogen atom.

**31.** The use according to claim 20, wherein $R^6$ is a hydrogen atom, an alkyl group having from 1 to 4 carbon atoms, a benzyl group or a phenethyl group.

**32.** The use according to claim 20, wherein $R^6$ is a hydrogen atom, a methyl group or a benzyl group.

**33.** The use according to claim 20, wherein $R^6$ is a hydrogen atom.

**34.** The use according to claim 20, wherein A is an alkylene group having from 2 to 4 carbon atoms which is unsubstituted or is substituted by a carboxy group or an alkoxycarbonyl group wherein the alkoxy group has from 1 to 4 carbon atoms.

**35.** The use according to claim 20, wherein A is an alkylene group having from 2 to 4 carbon atoms.

**36.** The use according to claim 20, wherein A is an ethylene group or a 1-methylethylene group.

**37.** The use according to claim 20, wherein:

$R^3$ is a hydrogen atom, an alkyl group having from 1 to 4 carbon atoms, a benzyl group or a phenethyl group;
$R^4$ and $R^5$ are the same or different and each is a hydrogen atom, an alkyl group group having from 1 to 4 carbon atoms, a phenylalkyl group wherein the alkyl group has from 1 to 4 carbon atoms and the phenyl group is unsubstituted or is substituted by a substituent selected from the group consisting of alkyl groups having from 1 to 4 carbon atoms, alkoxy groups having from 1 to 4 carbon atoms, hydroxy groups, halogen atoms and nitro groups, a naphthylmethyl group, a phenyl group which is unsubstituted or is substituted by a substituent selected from the group consisting of alkyl groups having from 1 to 4 carbon atoms, alkoxy groups having from 1 to 4 carbon atoms, hydroxy groups, halogen atoms and nitro groups, a naphthyl group or a heterocyclic group selected from furyl, thienyl, pyridyl, oxazolyl, thiazolyl, isoxazolyl and isothiazolyl groups, said group being unsubstituted or substituted by an alkyl group having from 1 to 4 carbon atoms;
$R^6$ is a hydrogen atom, an alkyl group having from 1 to 4 carbon atoms, a benzyl group or a phenethyl group; and
A is is an alkylene group having from 2 to 4 carbon atoms which is unsubstituted or is substituted by a carboxy group or an alkoxycarbonyl group wherein the alkoxy group has from 1 to 4 carbon atoms.

**38.** The use according to claim 20, wherein:

$R^3$ is a hydrogen atom, a methyl group or a benzyl group;
$R^4$ and $R^5$ are the same or different and each is a hydrogen atom, a methyl group, a benzyl group which is unsubstituted or is substituted by a substituent selected from the group consisting of methyl groups, methoxy groups, hydroxy groups, fluorine atoms and chlorine atoms, a phenethyl group which is unsubstituted or is substituted by a substituent selected from the group consisting of methyl groups, methoxy groups, hydroxy groups, fluorine atoms and chlorine atoms, a phenyl group which is unsubstituted or is substituted by a substituent selected from the group consisting of methyl groups, methoxy groups, hydroxy groups, fluorine atoms and chlorine atoms, a furyl group, a thienyl group or a pyridyl group;
$R^6$ is a hydrogen atom, a methyl group or a benzyl group; and
A is an alkylene group having from 2 to 4 carbon atoms.

**39.** The use according to claim 20, wherein:

W is a sulfur atom or an oxygen atom and X is a group having the formula $-NR^3-$, wherein $R^3$ is a hydrogen atom; or
X is a sulfur atom and W is a group having the formula $-NR^3-$, wherein $R^3$ is a hydrogen atom;
$R^4$ is a hydrogen atom, a methyl group, a benzyl group which is unsubstituted or is substituted by a substituent selected from the group consisting of methyl groups, methoxy groups and hydroxy groups, or a phenyl group which is unsubstituted or is substituted by a substituent selected from the group consisting of methyl groups, methoxy groups and hydroxy groups;
$R^5$ is a hydrogen atom;
$R^6$ is a hydrogen atom; and
A is an alkylene group having from 2 to 4 carbon atoms.

**40.** The use according to claim 20, wherein:

W is a sulfur atom or an oxygen atom and X is a group having the formula $-NR^3-$, wherein $R^3$ is a hydrogen atom;
$R^4$ is a hydrogen atom, a methyl group, a benzyl group, a phenyl group or a methoxyphenyl group;

$R^5$ is a hydrogen atom;
$R^6$ is a hydrogen atom; and
A is an alkylene group having from 2 to 4 carbon atoms.

**41.** The use according to claim 20, wherein:

W is a sulfur atom and X is a group having the formula $-NR^3-$, wherein $R^3$ is a hydrogen atom;
$R^4$ is a hydrogen atom, a methyl group, a benzyl group or a 4-methoxyphenyl group;
$R^5$ is a hydrogen atom;
$R^6$ is a hydrogen atom; and
A is an ethylene group or a 1-methylethylene group.

**42.** The use according to claim 20, wherein:

W is a sulfur atom and X is.a group having the formula -NH-;
$R^4$ is a hydrogen atom;
$R^5$ is a hydrogen atom;
$R^6$ is a hydrogen atom; and
A is an ethylene group or a 1-methylethylene group.

**43.** The use according to claim 20, wherein the thia- or oxazolidinone derivative or pharmacologically acceptable salt thereof is selected from the group consisting of:

N-(2-nitroxyethyl)-2-oxothiazolidin-4-ylcarboxamide;
N-(2-nitroxyethyl)-5-methyl-2-oxothiazolidin-4-ylcarboxamide;
N-(2-nitroxyethyl)-5-(4-methoxyphenyl)-2-oxothiazolidin-4-ylcarboxamide;
N-(2-nitroxyethyl)-5-benzyl-2-oxothiazolidin-4-ylcarboxamide;
N-(1-methyl-2-nitroxyethyl)-2-oxothiazolidin-4-ylcarboxamide;
N-(2-nitroxyethyl)-2-oxoxazolidin-4-ylcarboxamide;
N-(2-nitroxyethyl)-5-(3-furyl)-2-oxoxazolidin-4-ylcarboxamide;
N-(2-nitroxyethyl)-5-benzyl-2-oxoxazolidin-4-ylcarboxamide;
N-(1-methyl-2-nitroxyethyl)-2-oxoxazolidin-4-ylcarboxamide;
N-(1-methyl-2-nitroxyethyl)-5-methyl-2-oxothiazolidin-4-ylcarboxamide;
N-(-methyl-2-nitroxyethyl)-5-(4-methoxyphenyl)-2-oxothiazolidin-4-ylcarboxamide;
N-(1-methyl-2-nitroxyethyl)-5-benzyl-2-oxothiazolidin-4-ylcarboxamide;
N-(1-methyl-2-nitroxyethyl)-5-(3-furyl)-2-oxoxazolidin-4-ylcarboxamide;
N-(1-methyl-2-nitroxyethyl)-5-benzyl-2-oxoxazolidin-4-ylcarboxamide;
N-(2-nitroxyethyl)-2-oxothiazolidin-5-ylcarboxamide;
N-(2-nitroxyethyl)-4-(4-methoxyphenyl)-2-oxothiazolidin-5-ylcarboxamide;
N-(1-methyl-2-nitroxyethyl)-2-oxothiazolidin-5-ylcarboxamide; and
N-(1-methyl-2-nitroxyethyl)-4-(4-methoxyphenyl)-2-oxothiazolidin-5-ylcarboxamide; and pharmacologically acceptable salts thereof.

## Patentansprüche

**1.** Optisch aktives Thiazolidinon-Derivat der allgemeinen Formel

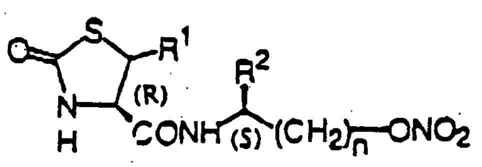

(I)

worin:

$R^1$ ein Wasserstoffatom, eine Alkylgruppe mit 1 bis 4 Kohlenstoffatomen, eine Phenylgruppe, die unsubstituiert ist oder die mit einem Substituenten substituiert ist, der aus der aus der Alkylgruppen mit 1 bis 4 Kohlenstoffatomen, Alkoxygruppen mit 1 bis 4 Kohlenstoffatomen und Halogenatomen bestehenden Gruppe ausgewählt ist, oder eine Phenylalkylgruppe, in der die Alkylgruppe 1 oder 2 Kohlenstoffatome hat und die Phenylgruppe unsubstituiert ist oder mit einem Substituenten substituiert ist, der aus der Gruppe der Alkylgruppen mit 1 bis 4 Kohlenstoffatomen, Alkoxygruppen mit 1 bis 4 Kohlenstoffatomen und Halogenatomen ausgewählt ist, darstellt,

$R^2$ eine Alkylgruppe mit 1 bis 6 Kohlenstoffatomen darstellt und

n 1 oder 2 darstellt.

**2.** Optisch aktives Thiazolidinon-Derivat nach Anspruch 1, worin $R^1$ ein Wasserstoffatom, eine Alkylgruppe mit 1 bis 4 Kohlenstoffatomen, eine Phenylgruppe, die unsubstituiert ist oder die mit einem unter Methylgruppen, Methoxygruppen, Fluoratomen und Chloratomen ausgewählten Substituenten substituiert ist, eine Benzylgruppe, die unsubstituiert ist oder die mit einem Substituenten substituiert ist, der unter Methylgruppen, Methoxygruppen, Fluoratomen und Chloratomen ausgewählt ist, oder eine Phenethylgruppe, die unsubstituiert ist oder die mit einem Substituenten substituiert ist, der unter Methylgruppen, Methoxygruppen, Fluoratomen und Chloratomen ausgewählt ist, darstellt.

**3.** Optisch aktives Thiazolidinon-Derivat nach Anspruch 1, worin $R^1$ ein Wasserstoffatom, eine Methylgruppe, eine 4-Methoxyphenylgruppe oder eine Benzylgruppe ist.

**4.** Optisch aktives Thiazolidinon-Derivat nach Anspruch 1, worin $R^1$ ein Wasserstoffatom ist.

**5.** Optisch aktives Thiazolidinon-Derivat nach Anspruch 1, worin $R^2$ eine Alkylgruppe mit 1 bis 4 Kohlenstoffatomen ist.

**6.** Optisch aktives Thiazolidinon-Derivat nach Anspruch 1, worin $R^2$ eine Methylgruppe, eine Ethylgruppe, eine Propylgruppe, eine Isopropylgruppe, eine Butylgruppe oder eine Isobutylgruppe ist.

**7.** Optisch aktives Thiazolidinon-Derivat nach Anspruch 1, worin $R^2$ eine Methylgruppe, eine Propylgruppe, eine Butylgruppe oder eine Isobutylgruppe ist.

**8.** Optisch aktives Thiazolidinon-Derivat nach Anspruch 1, worin $R^2$ eine Methylgruppe ist.

**9.** Optisch aktives Thiazolidinon-Derivat nach Anspruch 1, worin n 1 ist.

**10.** Optisch aktives Thiazolidinon-Derivat nach Anspruch 1, worin

$R^1$ ein Wasserstoffatom, eine Alkylgruppe mit 1 bis 4 Kohlenstoffatomen, eine Phenylgruppe, die unsubstituiert ist oder die mit einem unter Methylgruppen, Methoxygruppen, Fluoratomen und Chloratomen ausgewählten Substituenten substituiert ist, eine Benzylgruppe, die unsubstituiert ist oder die mit einem unter Methylgruppen, Methoxygruppen, Fluoratomen und Chloratomen ausgewählten Substituenten substituiert ist, oder eine Phenethylgruppe, die unsubstituiert ist oder die mit einem unter Methylgruppen, Methoxygruppen, Fluoratomen und Chloratomen ausgewählten Substituenten substituiert ist, darstellt,
$R^2$ eine Alkylgruppe mit 1 bis 4 Kohlenstoffatomen ist und n 1 ist.

**11.** Optisch aktives Thiazolidinon-Derivat nach Anspruch 1, worin

$R^1$ ein Wasserstoffatom, eine Methylgruppe, eine 4-Methoxyphenylgruppe oder eine Benzylgruppe ist,
$R^2$ eine Methylgruppe, eine Ethylgruppe, eine Propylgruppe, eine Isopropylgruppe, eine Butylgruppe oder eine Isobutylgruppe ist und
n 1 ist.

**12.** Optisch aktives Thiazolidinon-Derivat nach Anspruch 1, worin

$R^1$ ein Wasserstoffatom ist,

$R^2$ eine Methylgruppe, eine Propylgruppe, eine Butylgruppe oder eine Isobutylgruppe ist und n 1 ist.

**13.** Optisch aktives Thiazolidinon-Derivat nach Anspruch 1, worin $R^1$ ein Wasserstoffatom ist und $R^2$ eine Methylgruppe ist.

**14.** Optisch aktives Thiazolidinon-Derivat nach Anspruch 1, das aus der folgenden Gruppe ausgewählt ist:

(4R)-N-[(1S)-1-Methyl-2-nitroxyethyl]-2-oxothiazolidin-4-yl-carbonsäureamid,
(4R)-N-[(1S)-1-Methyl-2-nitroxyethyl]-5-methyl-2-oxothiazolidin-4-yl-carbonsäureamid,
(4R)-N-[(1S)-1-Methyl-2-nitroxyethyl]-5(4-methoxyphenyl)-2-oxothiazolidin-4-yl-carbonsäureamid,
(4R)-N-[(1S)-1-Methyl-2-nitroxyethyl] -5-benzyl-2-oxothiazolidin-4-yl-carbonsäureamid,
(4R)-N-[(1S)-1-Ethyl-2-nitroxyethyl]-2-oxothiazolidin-4-yl-carbonsäureamid,
(4R)-N-[(1S)-1-Propyl-2-nitroxyethyl] -2-oxothiazolidin-4-yl-carbonsäureamid,
(4R)-N-[(1S)-1-Propyl-2-nitroxyethyl]-5-methyl-2-oxothiazolidin-4-yl-carbonsäureamid,
(4R)-N-[(1S)-1-Propyl-2-nitroxyethyl]-5-(4-methoxyphenyl)-2-oxothiazolidin-4-yl-carbonsäureamid,
(4R)-N-[(1S)-1-Propyl-2-nitroxyethyl]-5-benzyl-2-oxothiazolidin-4-yl-carbonsäureamid,
(4R)-N-[(1S)-1-Butyl-2-nitroxyethyl]-2-oxothiazolidin-4-yl-carbonsäureamid,
(4R)-N-[(1S)-1-Butyl-2-nitroxyethyl]-5-methyl-2-oxothiazolidin-4-yl-carbonsäureamid,
(4R)-N-[(1S)-1-Butyl-2-nitroxyethyl]-5-(4-methoxyphenyl)-2-oxothiazolidin-4-yl-carbonsäureamid,
(4R)-N-[(1S)-1-Butyl-2-nitroxyethyl]-5-benzyl-2-oxothiazolidin-4-yl-carbonsäureamid,
(4R)-N-[(1S)-1-Isobutyl-2-nitroxyethyl]-2-oxothiazolidin-4-yl-carbonsäureamid,
(4R)-N-[(1S)-1-Isobutyl-2-nitroxyethyl]-5-methyl-2-oxothiazolidin-4-yl-carbonsäureamid,
(4R)-N-[(1S)-1-Isobutyl-2-nitroxyethyl]-5-(4-methoxyphenyl)-2-oxothiazolidin-4-yl-carbonsäureamid und
(4R)-N-[(1S)-1-Isobutyl-2-nitroxyethyl]-5-benzyl-2-oxothiazolidin-4-yl-carbonsäureamid.

**15.** Zusammensetzung zur Verhütung oder Behandlung von Angina pectoris, die eine wirksame Menge einer aktiven Verbindung im Gemisch mit einem pharmakologisch geeigneten Träger oder Verdünnungsmittel enthält, wobei die aktive Verbindung ein optisch aktives Thiazolidinon-Derivat nach einem Ansprüche 1 bis 14 ist.

**16.** Optisch aktives Thiazolidinon-Derivat nach einem der Ansprüche 1 bis 14 zur Verwendung als Arzneimittel.

**17.** Verwendung eines optisch aktiven Thiazolidinon-Derivats nach einem der Ansprüche 1 bis 14 zur Herstellung eines Arzneimittels zur Verhütung oder Behandlung von Angina pectoris.

**18.** Verfahren zur Herstellung eines optisch aktiven Thiazolidinon-Derivats nach einem der Ansprüche 1 bis 14, welches die Umsetzung einer Carbonsäure der allgemeinen Formel

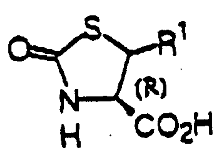

(III)

worin $R^1$ wie in Anspruch 1 definiert ist,
oder eines reaktiven Derivats dieser mit einem Amin der allgemeinen Formel

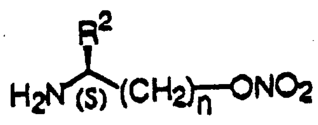

(IV)

worin $R^2$ wie in Anspruch 1 definiert ist, oder eines Säureadditionssalzes davon, umfaßt.

**19.** Verfahren zur Herstellung eines optisch aktiven Thiazolidinon-Derivats nach einem der Ansprüche 1 bis 14, welches die Umsetzung einer Carbonsäure der allgemeinen Formel

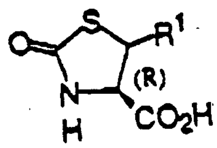

(III)

worin $R^1$ wie in Anspruch 1 definiert ist,
oder eines reaktiven Derivats dieser mit einer Aminverbindung der allgemeinen Formel

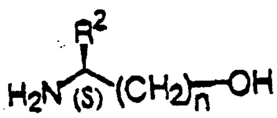

(V)

worin $R^2$ wie in Anspruch 1 definiert ist,
oder eines Säureadditionssalzes davon und nachfolgende Nitrierung der so erhaltenen Verbindung umfaßt.

**20.** Verwendung eines Thia- oder Oxazolidinon-Derivats der nachstehenden allgemeinen Formel (II) oder eines pharmakologisch geeigneten Salzes davon zur Herstellung eines Arzneimittels zur Behandlung oder Verhütung von ulcerativen Erkrankungen

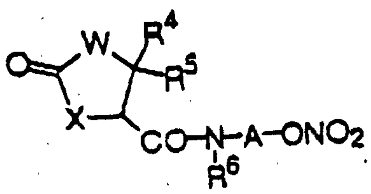

(II)

worin

W ein Schwefelatom oder ein Sauerstoffatom bedeutet und
X eine Gruppe der Formel $-N(R^3)-$ darstellt oder
X ein Schwefelatom oder ein Sauerstoffatom darstellt und
W eine Gruppe der Formel $-N(R^3)-$ darstellt,
$R^3$ ein Wasserstoffatom, eine Alkylgruppe mit 1 bis 6 Kohlenstoffatomen oder eine Aralkylgruppe, in der die Alkylgruppe 1 bis 4 Kohlenstoffatome hat und die Arylgruppe wie nachstehend definiert ist, darstellt,
$R^4$ und $R^5$ gleich oder verschieden sind und jeweils ein Wasserstoffatom, eine Alkylgruppe mit 1 bis 6 Kohlenstoffatomen, eine Aralkylgruppe, in der die Alkylgruppe 1 bis 4 Kohlenstoffatome enthält und die Arylgruppe wie nachstehend definiert ist, eine wie nachstehend definierte Arylgruppe, eine 5- oder 6-gliedrige aromatische heterocyclische Gruppe mit 1 bis 3 Heteroatomen, die aus der aus Stickstoffatomen, Sauerstoffatomen und Schwefelatomen bestehenden Gruppe ausgewählt sind, welche gegebenenfalls mit einem Benzolring oder einer 5- oder 6-gliedrigen aromatischen heterocyclischen Gruppe kondensiert sein kann, die 1 bis 3 Heteroatome enthält, die aus der aus Stickstoffatomen, Sauerstoffatomen und Schwefelatomen bestehenden Gruppe ausgewählt sind, die gegebenenfalls substituiert sein kann und gegebenenfalls mit einem Benzolring kondensiert sein kann, wobei die gegebenenfalls vorliegenden Substituenten aus der aus Alkylgruppen mit 1 bis 6 Kohlenstoffatomen, Aminogruppen und Mono- und Dialkylaminogruppen, wobei die oder jede Alkylgruppe 1 bis 6 Kohlenstoffatome enthält, bestehenden Gruppe ausgewählt sind, darstellen,
$R^6$ ein Wasserstoffatom, eine Alkylgruppe mit 1 bis 6 Kohlenstoffatomen oder eine Aralkylgruppe darstellt, wobei die Alkylgruppe 1 bis 4 Kohlenstoffatome enthält und die Arylgruppe wie nachstehend definiert ist, A

eine Alkylengruppe mit 2 bis 6 Kohlenstoffatomen darstellt, die unsubstituiert ist oder mit einem Substituenten substituiert ist, der aus der aus Carboxylgruppen, Alkoxycarbonylgruppen, deren Alkoxygruppen 1 bis 6 Kohlenstoffatome aufweisen und Aryloxycarbonylgruppen, in denen die Arylgruppe wie nachstehend definiert ist, bestehenden Gruppe ausgewählt ist,
wobei die vorstehend erwähnten Arylgruppen 6 bis 10 Kohlenstoffatome aufweisen und unsubstituiert sein können oder mit einem Substituenten substituiert sein können, der aus der aus Alkylgruppen mit 1 bis 6 Kohlenstoffatomen, Alkoxygruppen mit 1 bis 6 Kohlenstoffatomen, Hydroxygruppen, Halogenatomen, Aminogruppen, Mono- und Dialkylaminogruppen, in denen die oder jede Alkylgruppe 1 bis 6 Kohlenstoffatome aufweist, und Nitrogruppen bestehenden Gruppe ausgewählt ist.

**21.** Verwendung nach Anspruch 20, wobei $R^3$ ein Wasserstoffatom, eine Alkylgruppe mit 1 bis 4 Kohlenstoffatomen, eine Benzylgruppe oder eine Phenethylgruppe ist.

**22.** Verwendung nach Anspruch 20, wobei $R^3$ ein Wasserstoffatom, eine Methylgruppe oder eine Benzylgruppe ist.

**23.** Verwendung nach Anspruch 20, wobei

W ein Schwefelatom oder ein Sauerstoffatom ist und X eine Gruppe der Formel $-NR^3-$ ist , in der $R^3$ ein Wasserstoffatom ist, oder
X ein Schwefelatom und W eine Gruppe der Formel $-NR^3$ ist, worin $R^3$ ein Wasserstoffatom ist.

**24.** Verwendung nach Anspruch 20, wobei W ein Schwefelatom oder ein Sauerstoffatom ist und X eine Gruppe der Formel $-NR^3-$ ist, worin $R^3$ ein Wasserstoffatom ist.

**25.** Verwendung nach Anspruch 20, wobei $R^4$ und $R^5$ gleich oder verschieden sind und jeweils ein Wasserstoffatom, eine Alkylgruppe mit 1 bis 4 Kohlenstoffatomen, eine Phenylalkylgruppe, worin die Alkylgruppe 1 bis 4 Kohlenstoffatome hat und die Phenylgruppe unsubstituiert ist oder mit einem Substituenten substituiert ist, der aus der aus Alkylgruppen mit 1 bis 4 Kohlenstoffatomen, Alkoxygruppen mit 1 bis 4 Kohlenstoffatomen, Hydroxygruppen, Halogenatomen und Nitrogruppen bestehenden Gruppe ausgewählt ist, eine Naphthylmethylgruppe, eine Phenylgruppe, die unsubstituiert ist oder die mit einem Substituenten substituiert ist, der aus der aus Alkylgruppen mit 1 bis 4 Kohlenstoffatomen, Alkoxygruppen mit 1 bis 4 Kohlenstoffatomen, Hydroxygruppen, Halogenatomen und Nitrogruppen bestehenden Gruppe ausgewählt ist, eine Napthylgruppe oder eine heterocyclische Gruppe, die unter Furyl-, Thienyl-, Pyridyl-, Oxazolyl-, Thiazolyl-, Isoxazolyl- und Isothiazolylgruppen ausgewählt ist, wobei diese Gruppe unsubstituiert ist oder mit einer Alkylgruppe mit 1 bis 4 Kohlenstoffatomen substituiert ist, darstellen.

**26.** Verwendung nach Anspruch 20, wobei $R^4$ und $R^5$ gleich oder verschieden sind und jeweils ein Wasserstoffatom, eine Methylgruppe, eine Benzylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Phenethylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Phenylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Furylgruppe, eine Thienylgruppe oder eine Pyridylgruppe darstellen.

**27.** Verwendung nach Anspruch 20, wobei

$R^4$ ein Wasserstoffatom, eine Methylgruppe, eine Benzylgruppe, die unsubstituiert ist oder die mit einem unter Methylgruppen, Methoxygruppen und Hydroxygruppen ausgewählten Substituenten substituiert ist, oder eine Phenylgruppe, die unsubstituiert ist oder die mit einem unter Methylgruppen, Methoxygruppen und Hydroxygruppen ausgewählten Substituenten substituiert ist, darstellt und
$R^5$ ein Wasserstoffatom ist.

**28.** Verwendung nach Anspruch 20, wobei

$R^4$ ein Wasserstoffatom, eine Methylgruppe, eine Benzylgruppe, eine Phenylgruppe oder eine Methoxyphenylgruppe ist und
$R^5$ ein Wasserstoffatom ist.

**29.** Verwendung nach Anspruch 20, wobei

$R^4$ ein Wasserstoffatom, eine Methylgruppe, eine Benzylgruppe oder eine 4-Methoxyphenylgruppe ist und $R^5$ ein Wasserstoffatom ist.

**30.** Verwendung nach Anspruch 20, wobei $R^4$ ein Wasserstoffatom ist und $R^5$ ein Wasserstoffatom ist.

**31.** Verwendung nach Anspruch 20, wobei $R^6$ ein Wasserstoffatom, eine Alkylgruppe mit 1 bis 4 Kohlenstoffatomen, eine Benzylgruppe oder eine Phenethylgruppe ist.

**32.** Verwendung nach Anspruch 20, wobei $R^6$ ein Wasserstoffatom, eine Methylgruppe oder eine Benzylgruppe ist.

**33.** Verwendung nach Anspruch 20, wobei $R^6$ ein Wasserstoffatom ist.

**34.** Verwendung nach Anspruch 20, wobei A eine Alkylengruppe mit 2 bis 4 Kohlenstoffatomen ist, die unsubstituiert ist oder mit einer Carboxygruppe oder einer Alkoxycarbonylgruppe substituiert ist, worin die Alkoxygruppe 1 bis 4 Kohlenstoffatome aufweist.

**35.** Verwendung nach Anspruch 20, wobei A eine Alkylengruppe mit 2 bis 4 Kohlenstoffatomen ist.

**36.** Verwendung nach Anspruch 20, wobei A eine Ethylengruppe oder eine 1-Methylethylengruppe ist.

**37.** Verwendung nach Anspruch 20, wobei

$R^3$ ein Wasserstoffatom, eine Alkylgruppe mit 1 bis 4 Kohlenstoffatomen, eine Benzylgruppe oder eine Phenethylgruppe ist,
$R^4$ und $R^5$ gleich oder verschieden sind und jeweils ein Wasserstoffatom, eine Alkylgruppe mit 1 bis 4 Kohlenstoffatomen, eine Phenylalkylgruppe, worin die Alkylgruppe 1 bis 4 Kohlenstoffatome aufweist und die Phenylgruppe unsubstituiert ist oder mit einem Substituenten substituiert ist, der aus der aus Alkylgruppen mit 1 bis 4 Kohlenstoffatomen, Alkoxygruppen mit 1 bis 4 Kohlenstoffatomen, Hydroxygruppen, Halogenatomen und Nitrogruppen bestehenden Gruppe ausgewählt ist, eine Naphthylmethylgruppe, eine Phenylgruppe, die unsubstituiert ist oder die mit einem Substituenten substituiert ist, der aus der aus Alkylgruppen mit 1 bis 4 Kohlenstoffatomen, Alkoxygruppen mit 1 bis 4 Kohlenstoffatomen, Hydroxygruppen, Halogenatomen und Nitrogruppen bestehenden Gruppe ausgewählt ist, eine Naphthylgruppe oder eine heterocyclische Gruppe, die unter Furyl-, Thienyl-, Pyridyl-, Oxazolyl-, Thiazolyl-, Isoxazolyl- und Isothiazolylgruppen ausgewählt ist, wobei diese Gruppe unsubstituiert oder mit einer Alkylgruppe mit 1 bis 4 Kohlenstoffatomen substituiert ist, darstellen,
$R^6$ ein Wasserstoffatom, eine Alkylgruppe mit 1 bis 4 Kohlenstoffatomen, eine Benzylgruppe oder eine Phenethylgruppe ist und
A eine Alkylengruppe mit 2 bis 4 Kohlenstoffatomen, die unsubstituiert ist oder mit einer Carboxygruppe oder einer Alkoxycarbonylgruppe, worin die Alkoxygruppe 1 bis 4 Kohlenstoffatome enthält, substituiert ist, bedeutet.

**38.** Verwendung nach Anspruch 20, wobei

$R^3$ ein Wasserstoffatom, eine Methylgruppe oder eine Benzylgruppe ist,
$R^4$ und $R^5$ gleich oder verschieden sind und jeweils ein Wasserstoffatom, eine Methylgruppe, eine Benzylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Phenethylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Phenylgruppe, die unsubstituiert ist oder die mit einem Substituenten substituiert ist, der aus der aus Methylgruppen, Methoxygruppen, Hydroxygruppen, Fluoratomen und Chloratomen bestehenden Gruppe ausgewählt ist, eine Furylgruppe, eine Thienylgruppe oder eine Pyridylgruppe darstellen,
$R^6$ ein Wasserstoffatom, eine Methylgruppe oder eine Benzylgruppe ist und
A eine Alkylengruppe mit 2 bis 4 Kohlenstoffatomen ist.

**39.** Verwendung nach Anspruch 20, wobei

W ein Schwefelatom oder ein Sauerstoffatom ist und X eine Gruppe der Formel $-NR^3-$ ist, worin $R^3$ ein Wasserstoffatom darstellt, oder
X ein Schwefelatom ist und W eine Gruppe der Formel $-NR^3-$ ist, worin $R^3$ ein Wasserstoffatom darstellt,
$R^4$ ein Wasserstoffatom, eine Methylgruppe, eine Benzylgruppe, die unsubstituiert ist oder die mit einem Substituenten substituiert ist, der aus der aus Methylgruppen, Methoxygruppen und Hydroxygruppen bestehenden Gruppe ausgewählt ist, oder eine Phenylgruppe, die unsubstituiert ist oder die mit einem Substituenten substituiert ist, der aus der aus Methylgruppen, Methoxygruppen und Hydroxygruppen bestehenden Gruppe ausgewählt ist, darstellt,
$R^5$ ein Wasserstoffatom ist,
$R^6$ ein Wasserstoffatom ist und
A eine Alkylengruppe mit 2 bis 4 Kohlenstoffatomen ist.

**40.** Verwendung nach Anspruch 20, wobei

W ein Schwefelatom oder ein Sauerstoffatom ist und X eine Gruppe der Formel $-NR^3-$ ist, worin $R^3$ ein Wasserstoffatom darstellt,
$R^4$ ein Wasserstoffatom, eine Methylgruppe, eine Benzylgruppe, eine Phenylgruppe oder eine Methoxyphenylgruppe ist,
$R^5$ ein Wasserstoffatom ist,
$R^6$ ein Wasserstoffatom ist und
A eine Alkylengruppe mit 2 bis 4 Kohlenstoffatomen ist.

**41.** Verwendung nach Anspruch 20, wobei

W ein Schwefelatom ist und X eine Gruppe der Formel $-NR^3-$ ist, worin $R^3$ ein Wasserstoffatom darstellt,
$R^4$ ein Wasserstoffatom, eine Methylgruppe, eine Benzylgruppe oder eine 4-Methoxyphenylgruppe ist,
$R^5$ ein Wasserstoffatom ist,
$R^6$ ein Wasserstoffatom ist und
A eine Ethylengruppe oder eine 1-Methylethylengruppe ist.

**42.** Verwendung nach Anspruch 20, wobei

W ein Schwefelatom ist und X eine Gruppe der Formel -NH- ist
$R^4$ ein Wasserstoffatom ist,
$R^5$ ein Wasserstoffatom ist,
$R^6$ ein Wasserstoffatom ist und
A eine Ethylengruppe oder eine 1-Methylethylengruppe ist.

**43.** Verwendung nach Anspruch 20, wobei das Thia- oder Oxazolidinon-Derivat oder dessen pharmakologisch geeignetes Salz aus der Gruppe der folgenden Verbindungen ausgewählt ist:

N- (2-Nitroxyethyl) -2-oxothiazolidin-4-ylcarbonsäureamid,
N- (2-Nitroxyethyl) -5-methyl-2-oxothiazolidin-4-ylcarbonsäureamid,
N- (2-Nitroxyethyl)-5-(4-methoxyphenyl)-2-oxothiazolidin-4-ylcarbonsäureamid,
N-(2-Nitroxyethyl)-5-benzyl-2-oxothiazolidin-4-ylcarbonsäureamid,
N-(1-Methyl-2-nitroxyethyl)-2-oxothiazolidin-4-ylcarbonsäureamid,
N- (2-Nitroxyethyl) -2-oxoxazolidin-4-ylcarbonsäureamid,
N-(2-Nitroxyethyl)5-(3-furyl)-2-oxothiazolidin-4-ylcarbonsäureamid,
N-(2-Nitroxyethyl)-5-benzyl-2-oxothiazolidin-4-ylcarbonsäureamid,
N-(1-Methyl-2-nitroxyethyl)-2-oxoxazolidin-4-ylcarbonsäureamid,
N-(1-Methyl-2-nitroxyethyl)-5-methyl-2-oxothiazolidin-4-ylcarbonsäureamid,
N-(1-Methyl-2-nitroxyethyl)-5-(4-methoxyphenyl)-2-oxothiazolidin-4-ylcarbonsäureamid,
N-(1-Methyl-2-nitroxyethyl)-5-benzyl-2-oxothiazolidin-4-ylcarbonsäureamid,
N-(1-Methyl-2-nitroxyethyl)-5-(3-furyl)-2-oxothiazolidin-4-ylcarbonsäureamid,
N-(1-Methyl-2-nitroxyethyl)-5-benzyl-2-oxoxazolidin-4-ylcarbonsäureamid,
N- (2-Nitroxyethyl)-2-oxothiazolidin-5-ylcarbonsäureamid,
N-(2-Nitroxyethyl)-4-(4-methoxyphenyl)-2-oxothiazolidin-5-ylcarbonsäureamid,
N- (1-Methyl-2-nitroxyethyl)-2-oxothiazolidin-5-ylcarbonsäureamid,

N-(1-Methyl-2-nitroxyethyl)-4-(4-methoxyphenyl)-2-oxothiazolidin-5-ylcarbonsäureamid und pharmakologisch geeigneten Salzen davon.

## Revendications

**1.** Dérivé de thiazolidinone optiquement actif ayant la formule générale :

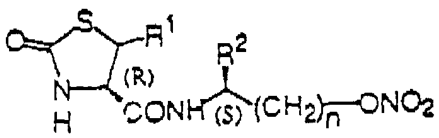

(I)

dans laquelle :

$R^1$ représente un atome d'hydrogène, un groupe alkyle ayant 1 à 4 atomes de carbone, un groupe phényle qui n'est pas substitué ou est substitué par un substituant choisi dans la classe formée par les groupes alkyle ayant 1 à 4 atomes de carbone, les groupes alcoxy ayant 1 à 4 atomes de carbone et les atomes d'halogène, ou un groupe phénylalkyle dans lequel le groupe alkyle compte 1 ou 2 atomes de carbone et le groupe phényle n'est pas substitué ou est substitué par un substituant choisi dans la classe formée par les groupes alkyle ayant 1 à 4 atomes de carbone, les groupes alcoxy ayant 1 à 4 atomes de carbone et les atomes d'halogène ;
$R^2$ représente un groupe alkyle ayant 1 à 6 atomes de carbone ; et
n représente 1 ou 2.

**2.** Dérivé de thiazolidinone optiquement actif selon la revendication 1, dans lequel $R^1$ est un atome d'hydrogène, un groupe alkyle ayant 1 à 4 atomes de carbone, un groupe phényle qui n'est pas substitué ou est substitué par un substituant choisi parmi les groupes méthyle, les groupes méthoxy, les atomes de fluor et les atomes de chlore, un groupe benzyle qui n'est pas substitué ou est substitué par un substituant choisi parmi les groupes méthyle, les groupes méthoxy, les atomes de fluor et les atomes de chlore, ou un groupe phénéthyle qui n'est pas substitué ou est substitué par un substituant choisi parmi les groupes méthyle, les groupes méthoxy, les atomes de fluor et les atomes de chlore.

**3.** Dérivé de thiazolidinone optiquement actif selon la revendication 1, dans lequel $R^1$ est un atome d'hydrogène, un groupe méthyle, un groupe 4-méthoxyphényle ou un groupe benzyle.

**4.** Dérivé de thiazolidinone optiquement actif selon la revendication 1, dans lequel $R^1$ est un atome d'hydrogène.

**5.** Dérivé de thiazolidinone optiquement actif selon la revendication 1, dans lequel $R^2$ est un groupe alkyle ayant 1 à 4 atomes de carbone.

**6.** Dérivé de thiazolidinone optiquement actif selon la revendication 1, dans lequel $R^2$ est un groupe méthyle, un groupe éthyle, un groupe propyle, un groupe isopropyle, un groupe butyle ou un groupe isobutyle.

**7.** Dérivé de thiazolidinone optiquement actif selon la revendication 1, dans lequel $R^2$ est un groupe méthyle, un groupe propyle, un groupe butyle ou un groupe isobutyle.

**8.** Dérivé de thiazolidinone optiquement actif selon la revendication 1, dans lequel $R^2$ est un groupe méthyle.

**9.** Dérivé de thiazolidinone optiquement actif selon la revendication 1, dans lequel n est 1.

**10.** Dérivé de thiazolidinone optiquement actif selon la revendication 1, dans lequel :

$R^1$ représente un atome d'hydrogène, un groupe alkyle ayant 1 à 4 atomes de carbone, un groupe phényle qui n'est pas substitué ou est substitué par un substituant choisi parmi les groupes méthyle, les groupes méthoxy, les atomes de fluor et les atomes de chlore, un groupe benzyle qui n'est pas substitué ou est substitué par un substituant choisi parmi les groupes méthyle, les groupes méthoxy, les atomes de fluor et les atomes

de chlore, ou un groupe phénéthyle qui n'est pas substitué ou est substitué par un substituant choisi parmi les groupes méthyle, les groupes méthoxy, les atomes de fluor et les atomes de chlore ;
$R^2$ est un groupe alkyle ayant 1 à 4 atomes de carbone ; et
n est 1.

**11.** Dérivé de thiazolidinone optiquement actif selon la revendication 1, dans lequel :

$R^1$ est un atome d'hydrogène, un groupe méthyle, un groupe 4-méthoxyphényle ou un groupe benzyle ;
$R^2$ est un groupe méthyle, un groupe éthyle, un groupe propyle, un groupe isopropyle, un groupe butyle ou un groupe isobutyle ; et
n est 1.

**12.** Dérivé de thiazolidinone optiquement actif selon la revendication 1, dans lequel :

$R^1$ est un atome d'hydrogène ;
$R^2$ est un groupe méthyle, un groupe propyle, un groupe butyle ou un groupe isobutyle ; et
n est 1.

**13.** Dérivé de thiazolidinone optiquement actif selon la revendication 1, dans lequel $R^1$ est un atome d'hydrogène et $R^2$ est un groupe méthyle.

**14.** Dérivé de thiazolidinone optiquement actif selon la revendication 1, choisi dans la classe formée par les suivants :

(4*R*)-N-[(1*S*)-1-méthyl-2-nitroxyéthyl]-2-oxothiazolidine-4-ylcarboxamide ;
(4*R*)-N-[(1*S*)-1-méthyl-2-nitroxyéthyl] -5-méthyl-2-oxothiazolidine-4-ylcarboxamide ;
(4*R*)-N-[(1*S*)-1-méthyl-2-nitroxyéthyl]-5-(4-méthoxyphényl)-2-oxothiazolidine-4-ylcarboxamide ;
(4*R*)-N-(1*S*)-1-méthyl-2-nitroxyéthyl]-5-benzyl-2-oxothiazolidine-4-ylcarboxamide ;
(4*R*)-N-(1*S*)-1-éthyl-2-nitroxyéthyl] -2-oxothiazolidine-4-ylcarboxamide ;
(4*R*)-N-[(1*S*)-1-propyl-2-nitroxyéthyl] -2-oxothiazolidine-4-ylcarboxamide ;
(4*R*)-N-[(1*S*)-1-propyl-2-nitroxyéthyl]-5-méthyl-2-oxothiazoldine-4-ylcarboxamide ;
(4*R*)-N-[(1*S*)-1-propyl-2-nitroxyéthyl]-5-(4-méthoxyphényl)-2-oxothiazolidine-4-ylcarboxamide ;
(4*R*)-N-[(1*S*)-1-propyl-2-nitroxyéthyl] -5-benzyl-2-oxothiazolidine-4-ylcarboxamide ;
(4*R*)-N-[(1*S*)-1-butyl-2-nitroxyéthyl] -2-oxothiazolidine-4-ylcarboxamide ;
(4*R*)-N-[(1*S*)-1-butyl-2-nitroxyéthyl] -5 -méthyl-2-oxothiazolidine-4-ylcarboxamide ;
(4*R*)-N-[(1*S*)-1-butyl-2-nitroxyéthyl]-5-(4-méthoxyphényl)-2-oxothiazolidine-4-ylcarboxamide ;
(4*R*)-N-[(1*S*)-1-butyl-2-nitroxyéthyl]-5-benzyl-2-oxothiazolidine-4-ylcarboxamide ;
(4*R*)-N-[(1*S*)-1-isobutyl-2-nitroxyéthyl] -2-oxothiazolidine-4-ylcarboxamide ;
(4*R*)-N-[(1*S*)-1-isobutyl-2-nitroxyéthyl] -5-méthyl-2-oxothiazolidine-4-ylcarboxamide ;
(4*R*)-N-[(1*S*)-1-isobutyl-2-nitroxyéthyl]-5-(4-méthoxyphényl)-2-oxothiazolidine-4-ylcarboxamide ; et
(4*R*)-N-[(1*S*)-1-isobutyl-2-nitroxyéthyl]-5-benzyl-2-oxothiazolidine-4-ylcarboxamide.

**15.** Composition destinée à prévenir ou traiter l'angine de poitrine, comprenant une quantité efficace d'un composé actif en mélange avec un support ou diluant pharmacologiquement acceptable, dans laquelle ledit composé actif est un dérivé de thiazolidinone optiquement actif selon l'une quelconque des revendications 1 à 14.

**16.** Dérivé de thiazolidinone optiquement actif selon l'une quelconque des revendications 1 à 14, pour son utilisation comme médicament.

**17.** Utilisation d'un dérivé de thiazolidinone optiquement actif selon l'une quelconque des revendications 1 à 14, dans la fabrication d'un médicament destiné à la prévention ou au traitement de l'angine de poitrine.

**18.** Procédé de préparation d'un dérivé de thiazolidinone optiquement actif selon l'une quelconque des revendications 1 à 14, comprenant la réaction d'un acide carboxylique ayant la formule générale :

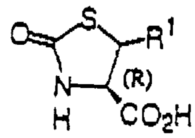

(III)

où $R^1$ est tel que défini dans la revendication 1, ou d'un dérivé réactif de celui-ci, avec une amine ayant la formule générale :

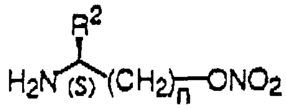

(IV)

où $R^2$ est tel que défini dans la revendication 1, ou un sel d'addition d'acide de celle-ci.

**19.** Procédé de préparation d'un dérivé de thiazolidinone optiquement actif selon l'une quelconque des revendications 1 à 14, comprenant la réaction d'un acide carboxylique ayant la formule générale :

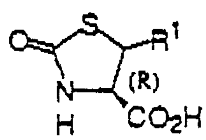

(III)

où $R^1$ est tel que défini dans la revendication 1, ou d'un dérivé réactif de celui-ci, avec une amine ayant la formule générale :

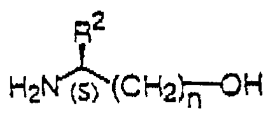

(V)

où $R^2$ est tel que défini dans la revendication 1, ou un sel d'addition d'acide de celle-ci, puis la nitration du composé ainsi obtenu.

**20.** Utilisation d'un dérivé de thia- ou oxazolidinone ayant la formule générale (II) ci-dessous, ou d'un sel pharmacologiquement acceptable de celui-ci, dans la fabrication d'un médicament destiné au traitement ou à la prévention d'une maladie ulcérative :

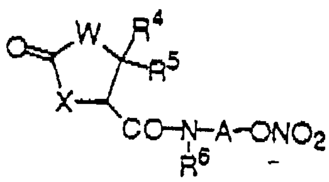

(II)

dans laquelle :

W représente un atome de soufre ou un atome d'oxygène et X représente un groupe ayant la formule $-N(R^3)-$,

ou
X représente un atome de soufre ou un atome d'oxygène et W représente un groupe ayant la formule -N($R^3$)- ; $R^3$ représente un atome d'hydrogène, un groupe alkyle ayant 1 à 6 atomes de carbone ou un groupe aralkyle dans lequel le groupe alkyle compte 1 à 4 atomes de carbone et le groupe aryle est tel que défini ci-dessous ; $R^4$ et $R^5$ sont identiques ou différents et représentent chacun un atome d'hydrogène, un groupe alkyle ayant 1 à 6 atomes de carbone, un groupe aralkyle dans lequel le groupe alkyle compte 1 à 4 atomes de carbone et le groupe aryle est tel que défini ci-dessous, un groupe aryle tel que défini ci-dessous, un groupe hétérocyclique aromatique à 5 ou 6 chaînons contenant 1 à 3 hétéroatomes choisis dans la classe formée par les atomes d'azote, les atomes d'oxygène et les atomes de soufre, qui peut facultativement être condensé avec un noyau benzénique ou un groupe hétérocyclique aromatique à 5 ou 6 chaînons contenant 1 à 3 hétéroatomes choisis dans la classe formée par les atomes d'azote, les atomes d'oxygène et les atomes de soufre qui peut être facultativement substitué et facultativement condensé avec un noyau benzénique, lesdits substituants facultatifs étant choisis dans la classe formée par les groupes alkyle ayant 1 à 6 atomes de carbone, les groupes amino et les groupes mono- et dialkylamino dans lesquels le ou chaque groupe alkyle compte 1 à 6 atomes de carbone ;
$R^6$ représente un atome d'hydrogène, un groupe alkyle ayant 1 à 6 atomes de carbone ou un groupe aralkyle dans lequel le groupe alkyle compte 1 à 4 atomes de carbone et le groupe aryle est tel que défini ci-dessous ;
A représente un groupe alkylène ayant 2 à 6 atomes de carbone et qui n'est pas substitué ou est substitué par un substituant choisi dans la classe formée par les groupes carboxyle, les groupes alcoxycarbonyle dans lesquels les groupes alcoxy ont 1 à 6 atomes de carbone et les groupes aryloxycarbonyle dans lesquels le groupe aryle est tel que défini ci-dessous ;
les groupes aryle susmentionnés ont 6 à 10 atomes de carbone et peuvent être non substitués ou peuvent être substitués par un substituant choisi dans la classe formée par les groupes alkyle ayant 1 à 6 atomes de carbone, les groupes alcoxy ayant 1 à 6 atomes de carbone, les groupes hydroxyle, les atomes d'halogène, les groupes amino, les groupes mono- et dialkylamino dans lesquels le ou chaque groupe alkyle compte 1 à 6 atomes de carbone et les groupes nitro.

**21.** Utilisation selon la revendication 20, dans laquelle $R^3$ est un atome d'hydrogène, un groupe alkyle ayant 1 à 4 atomes de carbone, un groupe benzyle ou un groupe phénéthyle.

**22.** Utilisation selon la revendication 20, dans laquelle $R^3$ est un atome d'hydrogène, un groupe méthyle ou un groupe benzyle.

**23.** Utilisation selon la revendication 20, dans laquelle :

W est un atome de soufre ou un atome d'oxygène, et X est un groupe ayant la formule -N$R^3$-, où $R^3$ est un atome d'hydrogène ; ou
X est un atome de soufre et W est un groupe ayant la formule -N$R^3$-, où $R^3$ est un atome d'hydrogène.

**24.** Utilisation selon la revendication 20, dans laquelle W est un atome de soufre ou un atome d'oxygène et X est un groupe ayant la formule -N$R^3$-, où $R^3$ est un atome d'hydrogène.

**25.** Utilisation selon la revendication 20, dans laquelle $R^4$ et $R^5$ sont identiques ou différents et chacun est un atome d'hydrogène, un groupe alkyle ayant 1 à 4 atomes de carbone, un groupe phénylalkyle dans lequel le groupe alkyle compte 1 à 4 atomes de carbone et le groupe phényle n'est pas substitué ou est substitué par un substituant choisi dans la classe formée par les groupes alkyle ayant 1 à 4 atomes de carbone, les groupes alcoxy ayant 1 à 4 atomes de carbone, les groupes hydroxyle, les atomes d'halogène et les groupes nitro, un groupe naphtylméthyle, un groupe phényle qui n'est pas substitué ou est substitué par un substituant choisi dans la classe formée par les groupes alkyle ayant 1 à 4 atomes de carbone, les groupes alcoxy ayant 1 à 4 atomes de carbone, les groupes hydroxyle, les atomes d'halogène et les groupes nitro, un groupe naphtyle ou un groupe hétérocyclique choisi parmi les groupes furyle, thiényle, pyridyle, oxazolyle, thiazolyle, isoxazolyle et isothiazolyle, ledit groupe n'étant pas substitué ou étant substitué par un groupe alkyle ayant 1 à 4 atomes de carbone.

**26.** Utilisation selon la revendication 20, dans laquelle $R^4$ et $R^5$ sont identiques ou différents et chacun est un atome d'hydrogène, un groupe méthyle, un groupe benzyle qui n'est pas substitué ou est substitué par un substituant choisi dans la classe formée par les groupes méthyle, les groupes méthoxy, les groupes hydroxyle, les atomes de fluor et les atomes de chlore, un groupe phénéthyle qui n'est pas substitué ou est substitué par un substituant choisi dans la classe formée par les groupes méthyle, les groupes méthoxy, les groupes hydroxyle, les atomes

de fluor et les atomes de chlore, un groupe phényle qui n'est pas substitué ou est substitué par un substituant choisi dans la classe formée par les groupes méthyle, les groupes méthoxy, les groupes hydroxyle, les atomes de fluor et les atomes de chlore, un groupe furyle, un groupe thiényle ou un groupe pyridyle.

**27.** Utilisation selon la revendication 20, dans laquelle :

$R^4$ est un atome d'hydrogène, un groupe méthyle, un groupe benzyle qui n'est pas substitué ou est substitué par un substituant choisi dans la classe formée par les groupes méthyle, les groupes méthoxy et les groupes hydroxyle, ou un groupe phényle qui n'est pas substitué ou est substitué par un substituant choisi dans la classe formée par les groupes méthyle, les groupes méthoxy et les groupes hydroxyle ; et
$R^5$ est un atome d'hydrogène.

**28.** Utilisation selon la revendication 20, dans laquelle :

$R^4$ est un atome d'hydrogène, un groupe méthyle, un groupe benzyle, un groupe phényle ou un groupe méthoxyphényle ; et
$R^5$ est un atome d'hydrogène.

**29.** Utilisation selon la revendication 20, dans laquelle :

$R^4$ est un atome d'hydrogène, un groupe méthyle, un groupe benzyle ou un groupe 4-méthoxyphényle ; et
$R^5$ est un atome d'hydrogène.

**30.** Utilisation selon la revendication 20, dans laquelle $R^4$ est un atome d'hydrogène et $R^5$ est un atome d'hydrogène.

**31.** Utilisation selon la revendication 20, dans laquelle $R^6$ est un atome d'hydrogène, un groupe alkyle ayant 1 à 4 atomes de carbone, un groupe benzyle ou un groupe phénéthyle.

**32.** Utilisation selon la revendication 20, dans laquelle $R^6$ est un atome d'hydrogène, un groupe méthyle ou un groupe benzyle.

**33.** Utilisation selon la revendication 20, dans laquelle $R^6$ est un atome d'hydrogène.

**34.** Utilisation selon la revendication 20, dans laquelle A est un groupe alkylène ayant 2 à 4 atomes de carbone qui n'est pas substitué ou est substitué par un groupe carboxyle ou un groupe alcoxycarbonyle dans lequel le groupe alcoxy compte 1 à 4 atomes de carbone.

**35.** Utilisation selon la revendication 20, dans laquelle A est un groupe alkylène ayant 2 à 4 atomes de carbone.

**36.** Utilisation selon la revendication 20, dans lequel A est un groupe éthylène ou un groupe 1-méthyléthylène.

**37.** Utilisation selon la revendication 20, dans laquelle :

$R^3$ est un atome d'hydrogène, un groupe alkyle ayant 1 à 4 atomes de carbone, un groupe benzyle ou un groupe phénéthyle ;
$R^4$ et $R^5$ sont identiques ou différents et chacun est un atome d'hydrogène, un groupe alkyle ayant 1 à 4 atomes de carbone, un groupe phénylalkyle dans lequel le groupe alkyle compte 1 à 4 atomes de carbone et le groupe phényle n'est pas substitué ou est substitué par un substituant choisi dans la classe formée par les groupes alkyle ayant 1 à 4 atomes de carbone, les groupes alcoxy ayant 1 à 4 atomes de carbone, les groupes hydroxyle, les atomes d'halogène et les groupes nitro, un groupe naphtylméthyle, un groupe phényle qui n'est pas substitué ou est substitué par un substituant choisi dans la classe formée par les groupes alkyle ayant 1 à 4 atomes de carbone, les groupes alcoxy ayant 1 à 4 atomes de carbone, les groupes hydroxyle, les atomes d'halogène et les groupes nitro, un groupe naphtyle ou un groupe hétérocyclique choisi parmi les groupes furyle, thiényle, pyridyle, oxazolyle, thiazolyle, isoxazolyle et isothiazolyle, ledit groupe n'étant pas substitué ou étant substitué par un groupe alkyle ayant 1 à 4 atomes de carbone ;
$R^6$ est un atome d'hydrogène, un groupe alkyle ayant 1 à 4 atomes de carbone, un groupe benzyle ou un groupe phénéthyle ; et
A est un groupe alkylène ayant 2 à 4 atomes de carbone qui n'est pas substitué ou est substitué par un groupe

carboxyle ou un groupe alcoxycarbonyle dans lequel le groupe alcoxy compte 1 à 4 atomes de carbone.

**38.** Utilisation selon la revendication 20, dans laquelle :

$R^3$ est un atome d'hydrogène, un groupe méthyle ou un groupe benzyle ;
$R^4$ et $R^5$ sont identiques ou différents et chacun est un atome d'hydrogène, un groupe méthyle, un groupe benzyle qui n'est pas substitué ou est substitué par un substituant choisi dans la classe formée par les groupes méthyle, les groupes méthoxy, les groupes hydroxyle, les atomes de fluor et les atomes de chlore, un groupe phénéthyle qui n'est pas substitué ou est substitué par un substituant choisi dans la classe formée par les groupes méthyle, les groupes méthoxy, les groupes hydroxyle, les atomes de fluor et les atomes de chlore, un groupe phényle qui n'est pas substitué ou est substitué par un substituant choisi dans la classe formée par les groupes méthyle, les groupes méthoxy, les groupes hydroxyle, les atomes de fluor et les atomes de chlore, un groupe furyle, un groupe thiényle ou un groupe pyridyle ;
$R^6$ est un atome d'hydrogène, un groupe méthyle ou un groupe benzyle ; et
A est un groupe alkylène ayant 2 à 4 atomes de carbone.

**39.** Utilisation selon la revendication 20, dans laquelle :

W est un atome de soufre ou un atome d'oxygène et X est un groupe ayant la formule $-NR^3-$, où $R^3$ est un atome d'hydrogène ; ou
X est un atome de soufre et W est un groupe ayant la formule $-NR^3-$, où $R^3$ est un atome d'hydrogène ;
$R^4$ est un atome d'hydrogène, un groupe méthyle, un groupe benzyle qui n'est pas substitué ou est substitué par un substituant choisi dans la classe formée par les groupes méthyle, les groupes méthoxy et les groupes hydroxyle, ou un groupe phényle qui n'est pas substitué ou est substitué par un substituant choisi dans la classe formée par les groupes méthyle, les groupes méthoxy et les groupes hydroxyle ;
$R^5$ est un atome d'hydrogène ;
$R^6$ est un atome d'hydrogène ; et
A est un groupe alkylène ayant 2 à 4 atomes de carbone.

**40.** Utilisation selon la revendication 20, dans laquelle :

W est un atome de soufre ou un atome d'oxygène et X est un groupe ayant la formule $-NR^3-$, où $R^3$ est un atome d'hydrogène ;
$R^4$ est un atome d'hydrogène, un groupe méthyle, un groupe benzyle, un groupe phényle ou un groupe méthoxyphényle ;
$R^5$ est un atome d'hydrogène ;
$R^6$ est un atome d'hydrogène ; et
A est un groupe alkylène ayant 2 à 4 atomes de carbone.

**41.** Utilisation selon la revendication 20, dans laquelle :

W est un atome de soufre et X est un groupe ayant la formule $-NR^3-$, où $R^3$ est un atome d'hydrogène ;
$R^4$ est un atome d'hydrogène, un groupe méthyle, un groupe benzyle ou un groupe 4-méthoxyphényle ;
$R^5$ est un atome d'hydrogène ;
$R^6$ est un atome d'hydrogène ; et
A est un groupe éthylène ou un groupe 1-méthyléthylène.

**42.** Utilisation selon la revendication 20, dans laquelle :

W est un atome de soufre et X est un groupe ayant la formule -NH- ;
$R^4$ est un atome d'hydrogène ;
$R^5$ est un atome d'hydrogène ;
$R^6$ est un atome d'hydrogène ; et
A est un groupe éthylène ou un groupe 1-méthyléthylène.

**43.** Utilisation selon la revendication 20, dans laquelle le dérivé de thia- ou oxazolidinone ou son sel pharmacologiquement acceptable est choisi dans la classe formée par les suivants :

N-(2-nitroxyéthyl) -2-oxothiazolidine-4-ylcarboxamide ;
N-(2-nitroxyéthyl)-5-méthyl-2-oxothiazolidine-4-ylcarboxamide ;
N-(2-nitroxyéthyl)-5-(4-méthoxyphényl)-2-oxothiazolidine-4-ylcarboxamide ;
N-(2-nitroxyéthyl)-5-benzyl-2-oxothiazolidine-4-ylcarboxamide ;
N-(1-méthyl-2-nitroxyéthyl)-2-oxothiazolidine-4-ylcarboxamide ;
N-(2-nitroxyéthyl) -2-oxoxazolidine-4-ylcarboxamide ;
N-(2-nitroxyéthyl) -5- (3-furyl) -2-oxoxazolidine-4-ylcarboxamide ;
N-(2-nitroxyéthyl)-5-benzyl-2-oxoxazolidine-4-ylcarboxamide ;
N-(1-méthyl-2-nitroxyéthyl) -2-oxoxazolidine-4-ylcarboxamide ;
N-(1-méthyl-2-nitroxyéthyl) -5-méthyl-2-oxothiazolidine-4-ylcarboxamide ;
N-(1-méthyl-2-nitroxyéthyl)-5-(4-méthoxyphényl)-2-oxothiazolidine-4-ylcarboxamide ;
N-(1-méthyl-2-nitroxyéthyl)-5-benzyl-2-oxothiazolidine-4-ylcarboxamide ;
N-(1-méthyl-2-nitroxyéthyl)-5-(3-furyl)-2-oxoxazolidine-4-ylcarboxamide ;
N-(1-méthyl-2-nitroxyéthyl) -5-benzyl-2-oxoxazolidine-4-ylcarboxamide ;
N-(2-nitroxyéthyl)-2-oxothiazolidine-5-ylcarboxamide ;
N-(2-nitroxyéthyl)-4-(4-méthoxyphényl)-2-oxothiazolidine-5-ylcarboxamide ;
N-(1-méthyl-2-nitroxyéthyl)-2-oxothiazolidine-5-yl-carboxamide ; et
N-(1-méthyl-2-nitroxyéthyl)-4-(4-méthoxyphényl)-2-oxo-thiazolidine-5-ylcarboxamide ;

et leurs sels pharmacologiquement acceptables.